(19) 

Europäisches Patentamt
European Patent Office
Office européen des brevets

(11) **EP 1 844 697 A1**

(12) **EUROPEAN PATENT APPLICATION**

(43) Date of publication:
   **17.10.2007 Bulletin 2007/42**

(51) Int Cl.:
   ***A61B 1/05*** (2006.01)

(21) Application number: **07005680.9**

(22) Date of filing: **20.03.2007**

(84) Designated Contracting States:
   **AT BE BG CH CY CZ DE DK EE ES FI FR GB GR HU IE IS IT LI LT LU LV MC MT NL PL PT RO SE SI SK TR**
   Designated Extension States:
   **AL BA HR MK YU**

(30) Priority: **23.03.2006 JP 2006081276**

(71) Applicant: **OLYMPUS MEDICAL SYSTEMS CORP. Tokyo (JP)**

(72) Inventor: **Kaneko, Kazuma Tokyo 151-0072 (JP)**

(74) Representative: **von Hellfeld, Axel Wuesthoff & Wuesthoff Patent- und Rechtsanwälte Schweigerstrasse 2 81541 München (DE)**

(54) **Image processing device**

(57)   An image processing device according to the present invention includes image capturing means for outputting an image capture signal based on a captured image of a subject, one or a plurality of storage means for storing the image capture signal, writing signal generation means for outputting to the storage means a writing signal for writing the image capture signal onto the storage means, switching signal generation means for outputting a switching signal for switching between a first and second observation modes, image operation means for performing an instruction about an operation with respect to at least one observation image in the first observation mode and the second observation mode, image operation invalidation means for setting an inoperative time for invalidating the instruction based on the switching signal, and image operation invalidation release means for releasing the invalidation after the switching signal is outputted and the inoperative time has passed.

**FIG.1**

EP 1 844 697 A1

**Description**

BACKGROUND OF THE INVENTION

1. Field of the Invention

The present invention relates to an image processing device, and more particularly, relates to an image processing device capable of switching a plurality of observation modes.

2. Description of the Related Art

Conventionally, endoscope apparatuses that have a light source device and an image processing device as essential parts have been widely used in medical fields. Particularly, in the medical fields, the endoscope apparatuses are mainly used when users inspect or observe within an organism.

As an example of the observation using the endoscope apparatus in the medical fields, other than an ordinary observation in which an image of the inside of an organism substantially similar to that observed with the naked eye is captured by irradiating white light in the organism, a fluorescence observation has been generally known. In the fluorescence observation, when excitation light that has a certain waveband is irradiated in an organism, a self-fluorescent image of a living tissue in the organism is captured, and the self fluorescent image is observed to determine a normal part and an affected part in the organism.

Further, in the observation using the endoscope apparatus in the medical fields, for example, a narrow band imaging (NBI) has been known. In the NBI, narrow band light that has a narrower band than irradiation light in ordinary observations is irradiated in an organism for observation. With the NBI, a blood vessel in a superficial portion of a mucous membrane can be observed with good contrast.

Further, in the observation using the endoscope apparatus in the medical fields, for example, an infrared observation has been known. In the infrared observation, near-infrared light that has a near-infrared band is irradiated in an organism for observation. In the infrared observation, a medical agent called indocyanine green (ICG) that has a characteristic to absorb light of near-infrared band is injected into a blood vessel so that hemodynamics of a lower deep portion of the mucous membrane where cannot be observed in the ordinary observation can be observed.

In an image processing apparatus proposed in Japanese Unexamined Patent Application Publication No. 2005-013611, the above-mentioned four observation modes, that is, the ordinary observation, the fluorescence observation, the NBI, and the infrared observation, can be switched and executed.

In the above-described fluorescence observation, the self-fluorescence of the living tissue in the organism is weak. Accordingly, the capturing of the self-fluorescent image of the living tissue in the organism is performed, for example, by reducing a rotation speed of a rotation filter installed in the light source device as compared with that in the ordinary observation to lengthen an exposure time as compared with that in the ordinary observation. Then, for example, while an observation mode in an endoscope apparatus is switched from the ordinary observation to the fluorescence observation, that is, the rotation speed of the rotation filter is changed from a rotation speed suitable for the ordinary observation to a rotation speed suitable for the fluorescence observation, a still image not suitable for recording is outputted. In Japanese Unexamined Patent Application Publication No. 2005-013611, any solution to the above-described problem is not proposed.

The present invention has been made in view of the above, and an object of the present invention is to provide an image processing device capable of outputting a still image suitable for recording in a case that an observation mode is switched from an observation mode to another observation mode.

SUMMARY OF THE INVENTION

A first image processing device according to the present invention includes image capturing means for capturing an image of a subject and outputting an image capture signal based on the captured image of the subject, one or a plurality of storage means for storing the image capture signal outputted from the image capturing means, writing signal generation means for outputting to the storage means a writing signal for writing the image capture signal onto the storage means, switching signal generation means for outputting to at least one of the image capturing means and the storage means a switching signal for switching between a first observation mode for creating a first observation image based on the image capture signal outputted from the image capturing means and a second observation mode for creating a second observation image different from the first observation image based on the image capture signal outputted from the image capturing means, image operation means for performing an instruction about an operation with respect to at least one of the first observation image and the second observation image, image operation invalidation means for setting an inoperative time for invalidating the instruction about the operation with respect to the one observation image based on the switching signal within a predetermined period of time, and image operation invalidation release means for releasing the invalidation after the switching signal is outputted and the inoperative time has passed.

A second image processing device according to the present invention includes image capturing means for capturing an image of a subject and outputting an image capture signal based on the captured image of the subject, one or a plurality of storage means for storing the image capture signal outputted from the image capturing means, writing signal generation means for outputting to the storage means a writing signal for writing the image capture signal onto the storage means, switching signal generation means for outputting to at least one of the image capturing means and the storage means a switching signal for switching between a first observation mode for creating a first observation image based on the image capture signal outputted from the image capturing means and a second observation mode for creating a second observation image different from the first observation image based on the image capture signal outputted from the image capturing means, writing forbidding means for stopping the writing of the image capture signal onto the storage means by stopping the output of the writing signal according to the switching signal, and writing forbiddance release means for releasing the stop of the writing of the image capture signal onto the storage means by resuming the output of the writing signal to the storage means after the switching signal is outputted and a predetermined period of time has passed.

A third image processing device according to the present invention, in the second image processing device, further includes freeze image creation means having the storage means, the freeze image creation means being configured to create a still image based on the image capture signal written on the storage means, and freeze instruction means for performing a freeze instruction for creating the still image to the freeze image creation means. The freeze image creation means invalidates the freeze instruction performed in the freeze instruction means for the predetermined period of time.

A fourth image processing device according to the present invention, in the second image processing device, further includes observation mode switching time setting means for setting the predetermined period of time.

A fifth image processing device according to the present invention, in the second image processing device, further includes information storage means on which certain information about at least a configuration of the image capturing means is written, and the predetermined period of time is set based on the certain information.

A sixth image processing device according to the present invention, in the third image processing device, the freeze image creation means further performs processing for extracting a plurality of still images including a least color shifted still image out of still images according to the image capture signal written on the storage means.

A seventh image processing device according to the present invention, in the first image processing device, further includes freeze image creation means having the storage means, the freeze image creation means being configured to perform processing for extracting a plurality of still images including a least color shifted still image out of still images according to the image capture signal written on the storage means; and freeze instruction means for performing a freeze instruction for creating the plurality of still images extracted by the freeze image creation means to the freeze image creation means. The freeze image creation means invalidates the processing in a case that the freeze instruction is performed in the freeze instruction means within the predetermined period of time except for the inoperative time.

A eighth image processing device according to the present invention, in the first image processing device, in the first observation image created in the first observation mode and the second observation image created in the second observation mode, one observation image denotes an image substantially similar to an image of the subject being observed with the naked eye, and another observation image denotes an image corresponding to an image of fluorescence generated by the subject.

A ninth image processing device according to the present invention, in the second image processing device, in the first observation image created in the first observation mode and the second observation image created in the second observation mode, one observation image denotes an image substantially similar to an image of the subject being observed with the naked eye, and another observation image denotes an image corresponding to an image of fluorescence generated by the subject.

A tenth image processing device according to the present invention, in the third image processing device, in the first observation image created in the first observation mode and the second observation image created in the second observation mode, one observation image denotes an image substantially similar to an image of the subject being observed with the naked eye, and another observation image denotes an image corresponding to an image of fluorescence generated by the subject.

An eleventh image processing device according to the present invention, in the fourth image processing device, in the first observation image created in the first observation mode and the second observation image created in the second observation mode, one observation image denotes an image substantially similar to an image of the subject being observed with the naked eye, and another observation image denotes an image corresponding to an image of fluorescence generated by the subject.

A twelfth image processing device according to the present invention, in the fifth image processing device, in the first observation image created in the first observation mode and the second observation image created in the second observation mode, one observation image denotes an image substantially similar to an image of the subject being observed with the naked eye, and another observation image denotes an image corresponding to an image of fluorescence generated by the subject.

A thirteenth image processing device according to the present invention, in the sixth image processing device, in the first observation image created in the first observation mode and the second observation image created in the second observation mode, one observation image denotes an image substantially similar to an image of the subject being observed with the naked eye, and another observation image denotes an image corresponding to an image of fluorescence generated by the subject.

A fourteenth image processing device according to the present invention, in the seventh image processing device, in the first observation image created in the first observation mode and the second observation image created in the second observation mode, one observation image denotes an image substantially similar to an image of the subject being observed with the naked eye, and another observation image denotes an image corresponding to an image of fluorescence generated by the subject.

A fifteenth image processing device according to the present invention, in the first image processing device, further includes an endoscope including an elongated insertion portion, and the image capturing means is provided in a tip part of the insertion portion.

A sixteenth image processing device according to the present invention, in the second image processing device, further includes an endoscope including an elongated insertion portion, and the image capturing means is provided in a tip part of the insertion portion.

A seventeenth image processing device according to the present invention, in the third image processing device, further includes an endoscope including an elongated insertion portion, and the image capturing means is provided in a tip part of the insertion portion.

A eighteenth image processing device according to the present invention, in the fourth image processing device, further includes an endoscope including an elongated insertion portion, and the image capturing means is provided in a tip part of the insertion portion.

A nineteenth image processing device according to the present invention, in the fifth image processing device, further includes an endoscope including an elongated insertion portion, and the image capturing means is provided in a tip part of the insertion portion.

A twentieth image processing device according to the present invention, in the sixth image processing device, further includes an endoscope including an elongated insertion portion, and the image capturing means is provided in a tip part of the insertion portion.

A twenty first image processing device according to the present invention, in the seventh image processing device, further includes an endoscope including an elongated insertion portion, and the image capturing means is provided in a tip part of the insertion portion.

BRIEF DESCRIPTION OF THE DRAWINGS

Fig. 1 is a view illustrating essential parts of an endoscope device according to an embodiment of the present invention;
Fig. 2 is a view illustrating an internal configuration of the endoscope device according to the embodiment of the present invention;
Fig. 3 is a view illustrating a configuration of a rotation filter provided in a light source section in the endoscope device according to the embodiment of the present invention;
Fig. 4 is a view illustrating transmission characteristics of an RGB filter provided in the rotation filter shown in Fig. 3;
Fig. 5 is a view illustrating transmission characteristics of a fluorescence observation filter provided in the rotation filter shown in Fig. 3;
Fig. 6 is a view illustrating a configuration of a band switching filter provided in the light source section in the endoscope device according to the embodiment of the present invention;
Fig. 7 is a view illustrating transmission characteristics of an ordinary/fluorescence observation filter and an infrared light observation filter provided in the band switching filter shown in Fig. 6;
Fig. 8 is a view illustrating transmission characteristics of a NBI filter provided in the band switching filter shown in Fig. 6;
Fig. 9 is a view illustrating transmission characteristics of an excitation light cut filter provided in an electronic endoscope in the endoscope device according to the embodiment of the present invention;
Fig. 10 is a view illustrating an example of setting screens of a processor provided in the endoscope device according to the embodiment of the present invention;

Fig. 11 is a view illustrating an example of configurations of an image capturing section provided in the electronic endoscope in the endoscope device according to the embodiment of the present invention;

Fig. 12 is a view illustrating an example different from the example shown in Fig. 11 illustrating a configuration of the image capturing section provided in the electronic endoscope in the endoscope device according to the embodiment of the present invention;

Fig. 13 is a flowchart illustrating an example of processing performed in the processor in a case that an observation mode is switched from an observation mode to another mode in the endoscope device according to the embodiment of the present invention;

Fig. 14 is a view illustrating an example of writing and readout states of an image capture signal in a memory section in a case that the observation mode is switched from an observation mode to another mode in the endoscope device according to the embodiment of the present invention;

Fig. 15 is a view illustrating an example different from the example shown in Fig. 10 illustrating a setting screen of the processor provided in the endoscope device according to the embodiment of the present invention;

Fig. 16 is a flowchart illustrating an example different from the example shown in Fig. 13 illustrating processing performed in the processor in a case that the observation mode is switched from an observation mode to another mode in the endoscope device according to the embodiment of the present invention;

Fig. 17 is a view illustrating an example of pre-freeze processing performed in the processor provided in the endoscope device according to the embodiment of the present invention;

Fig. 18 is a view illustrating an example of writing and readout states of an image capture signal in a synchronization circuit in a case that the observation mode is switched from an observation mode to another mode in the endoscope device according to the embodiment of the present invention;

Fig. 19 is a view illustrating an example different from the example shown in Fig. 18 illustrating a writing and readout state of the image capture signal in the synchronization circuit in the case that the observation mode is switched from the observation mode to another mode in the endoscope device according to the embodiment of the present invention;

Fig. 20 is a view illustrating an example different from the examples shown in Figs. 18 and 19 illustrating a writing and readout state of the image capture signal in the synchronization circuit in the case that the observation mode is switched from an observation mode to another mode in the endoscope device according to the embodiment of the present invention;

Fig. 21 is a view illustrating an example different from the example shown in Fig. 14 illustrating a writing and readout state of the image capture signal in the memory section in the case that the observation mode is switched from an observation mode to another mode in the endoscope device according to the embodiment of the present invention;

Fig. 22 is a schematic view illustrating another example of the pre-freeze processing performed in the processor provided in the endoscope device according to the embodiment of the present invention; and

Fig. 23 is a schematic view illustrating processing to be performed concomitantly with the processing shown in Fig. 22 in the processor provided in the endoscope device according to the embodiment of the present invention.

DESCRIPTION OF THE PREFERRED EMBODIMENTS

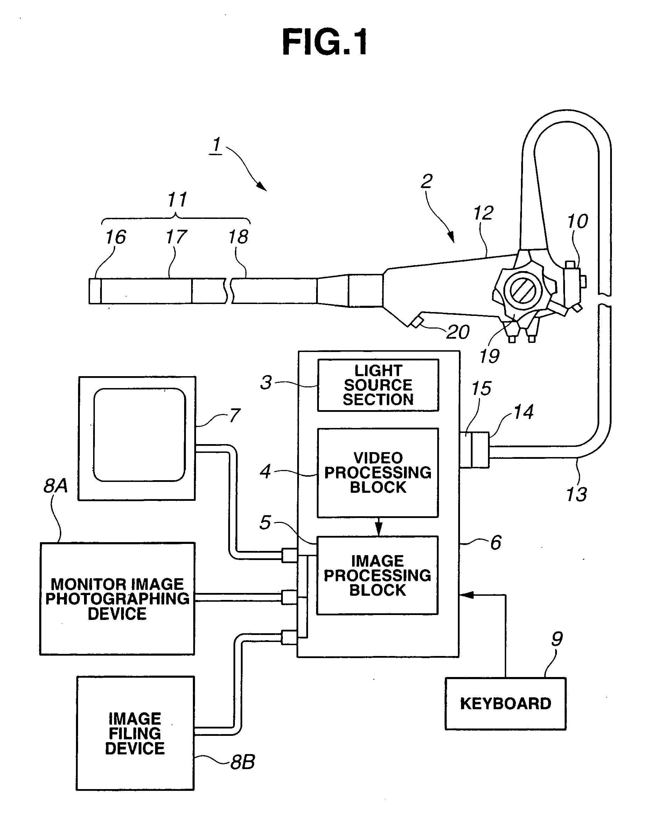

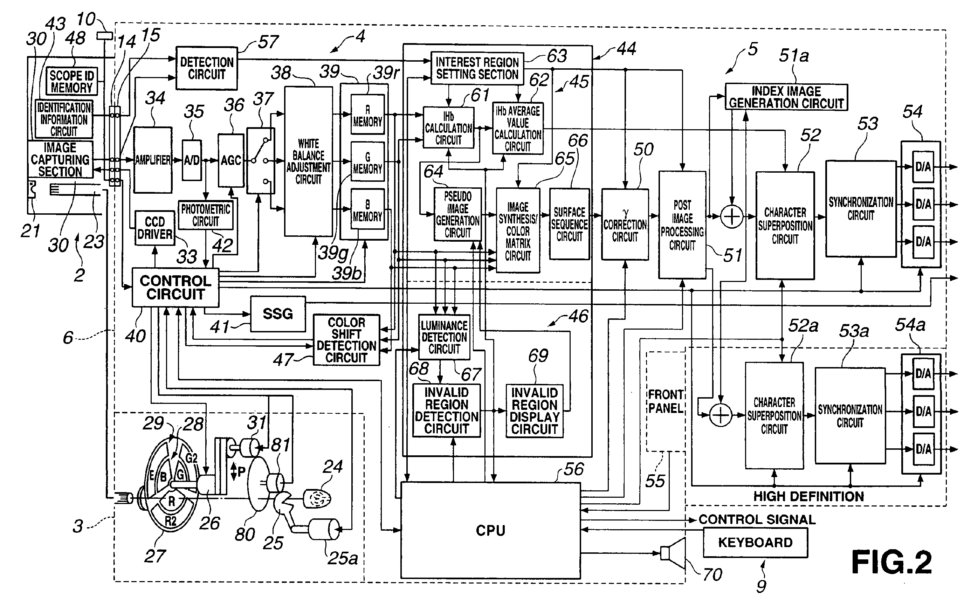

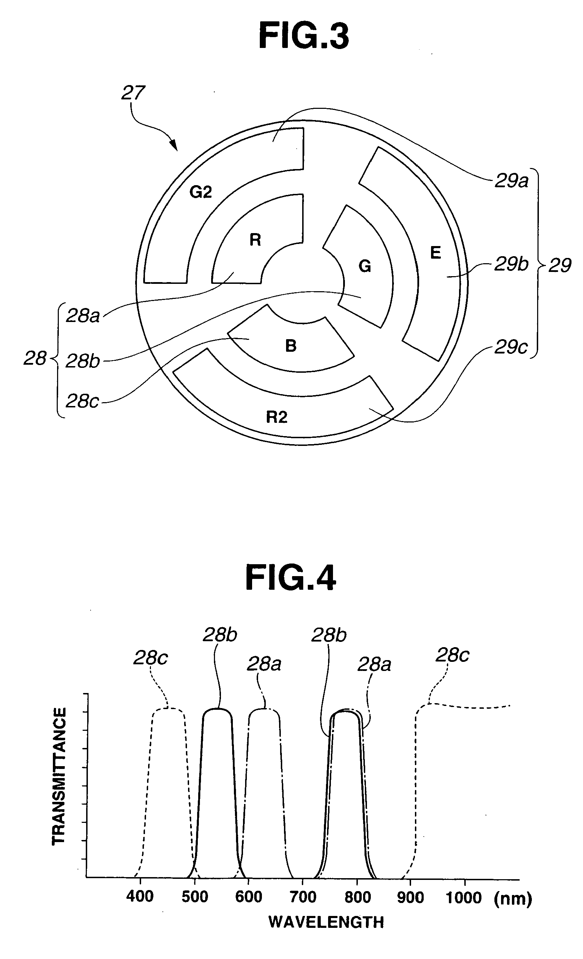

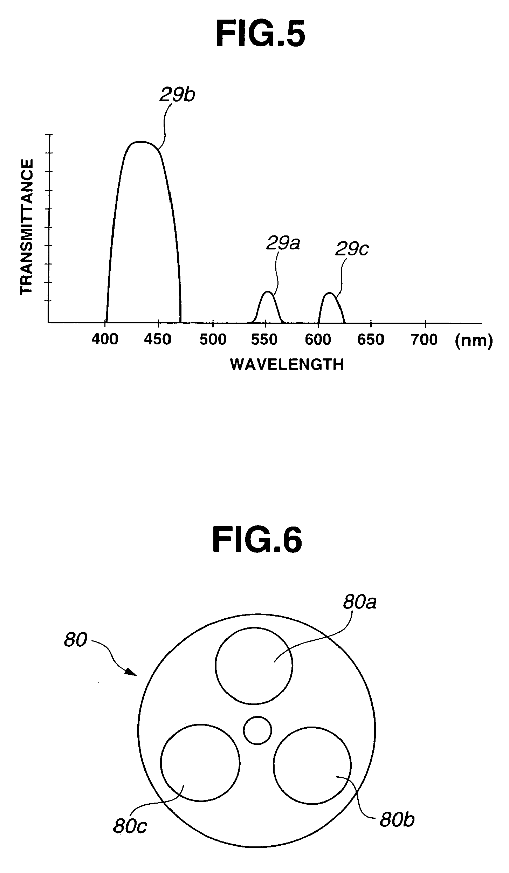

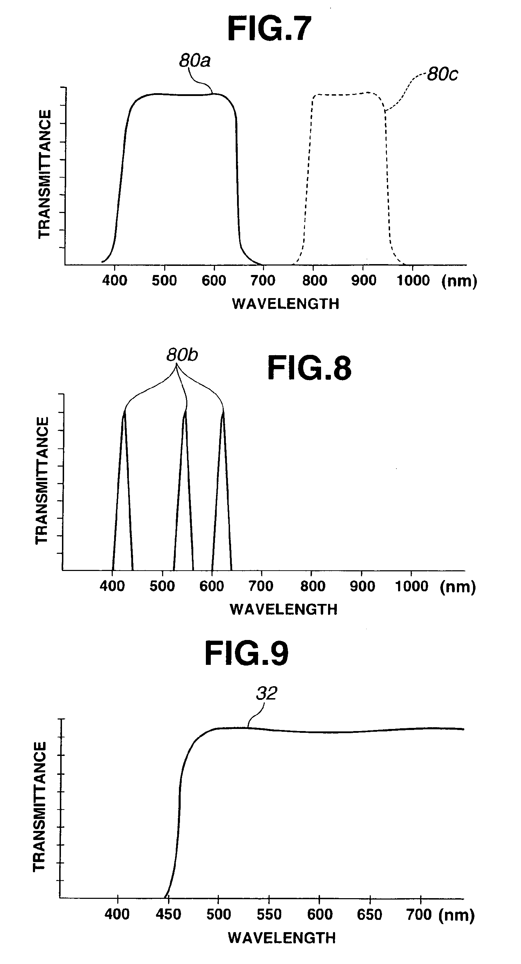

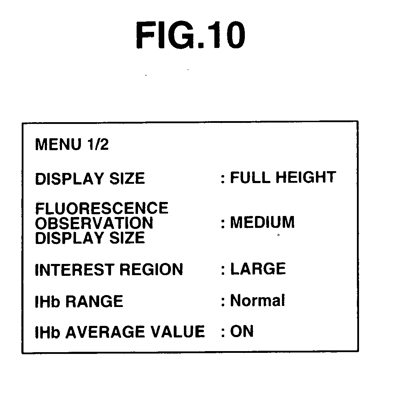

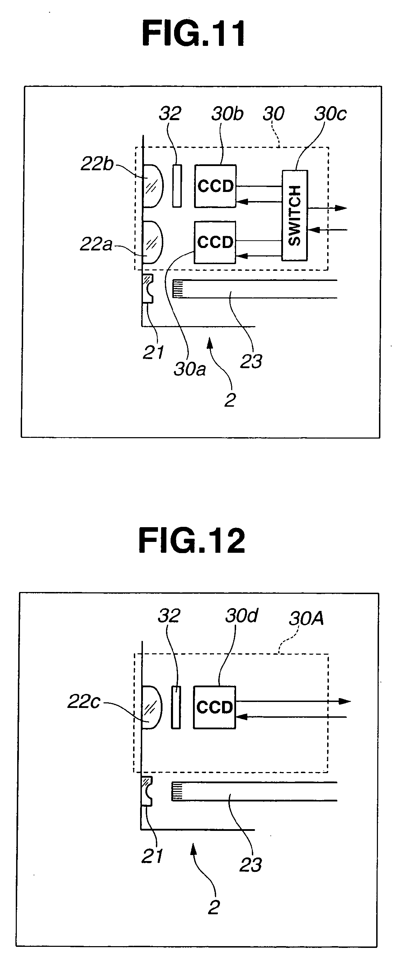

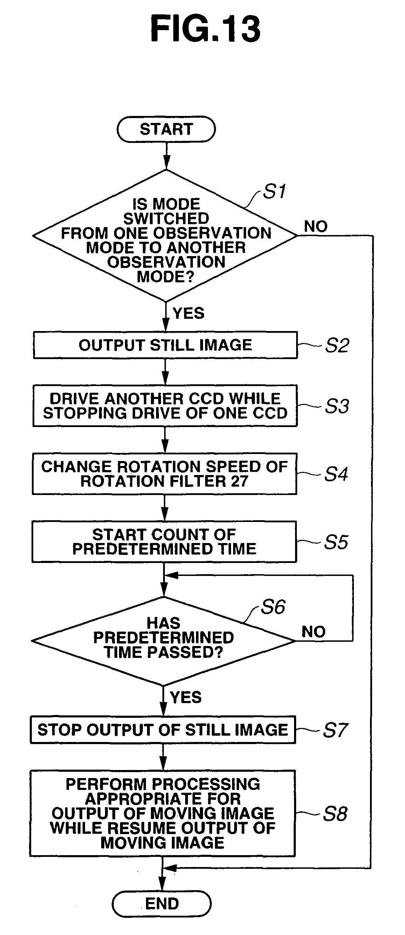

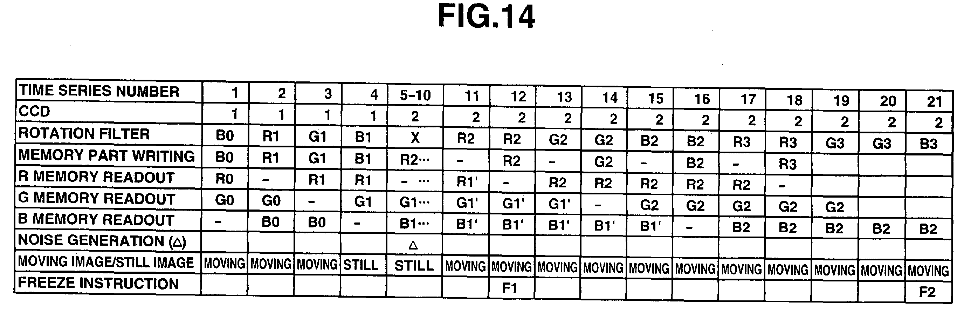

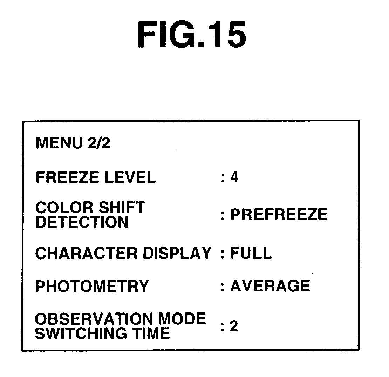

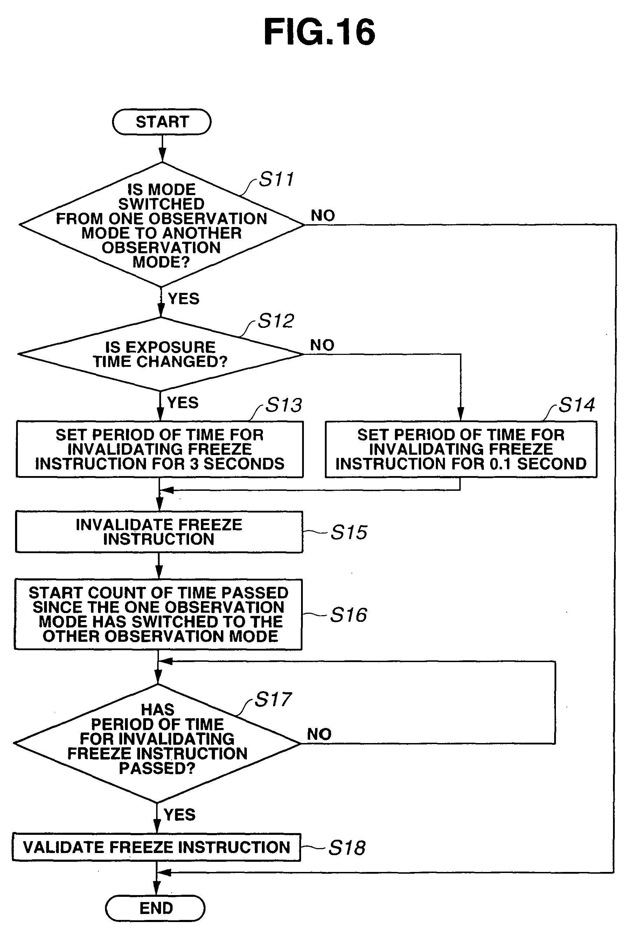

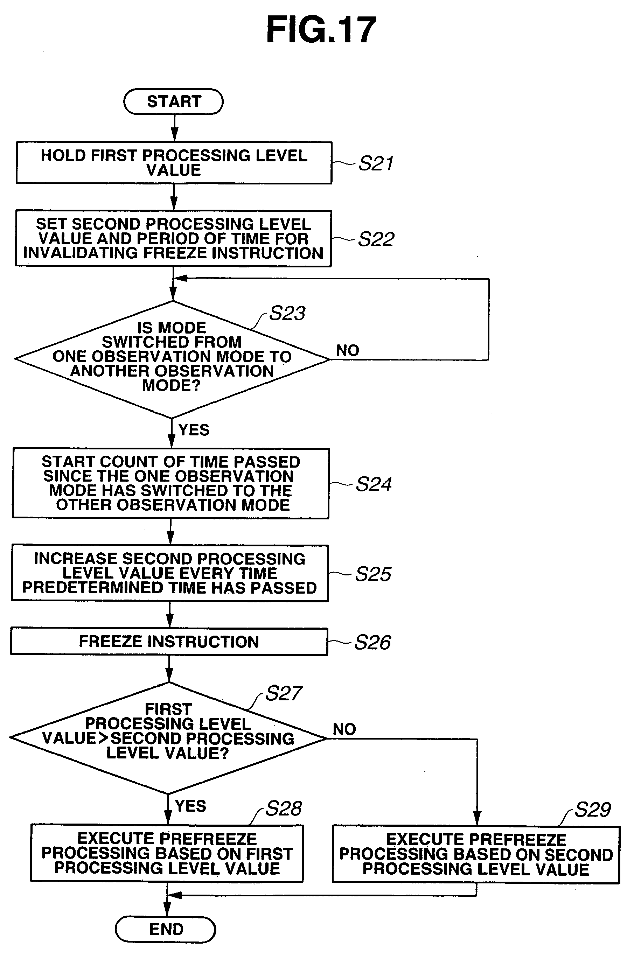

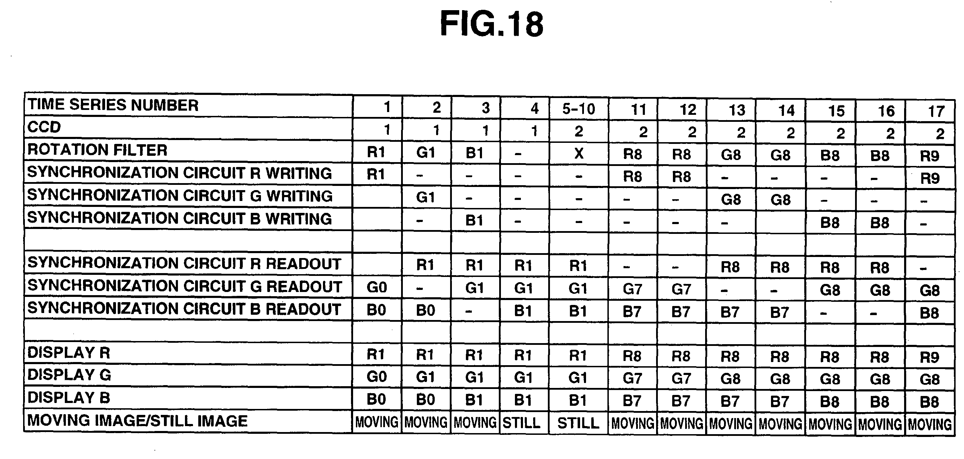

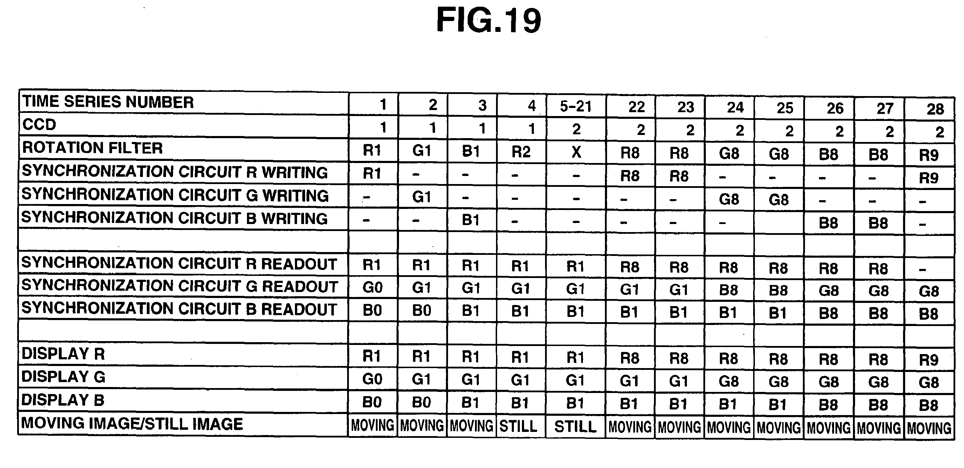

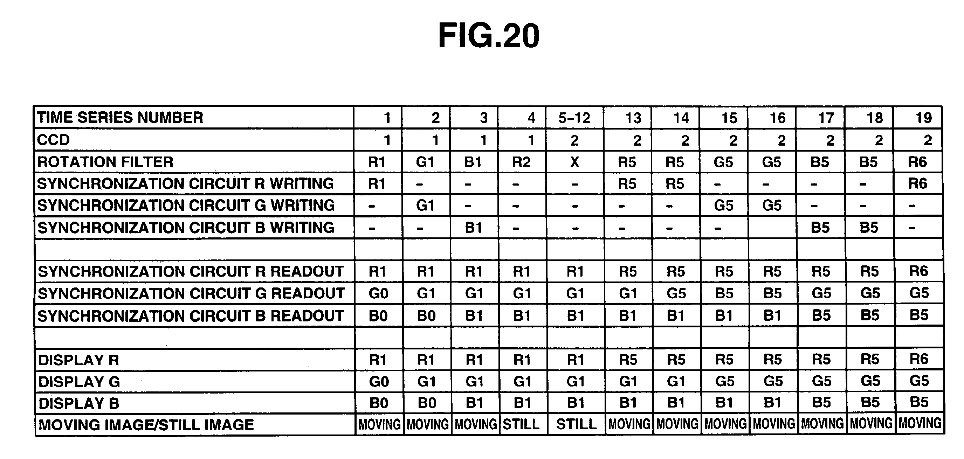

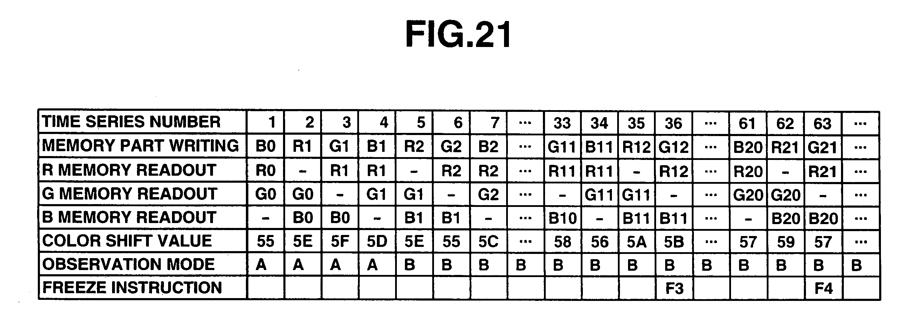

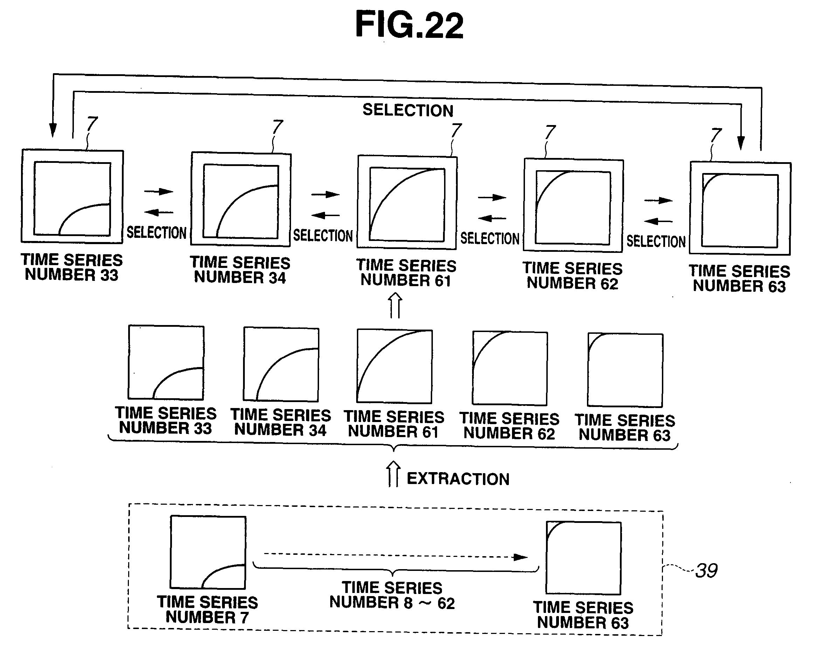

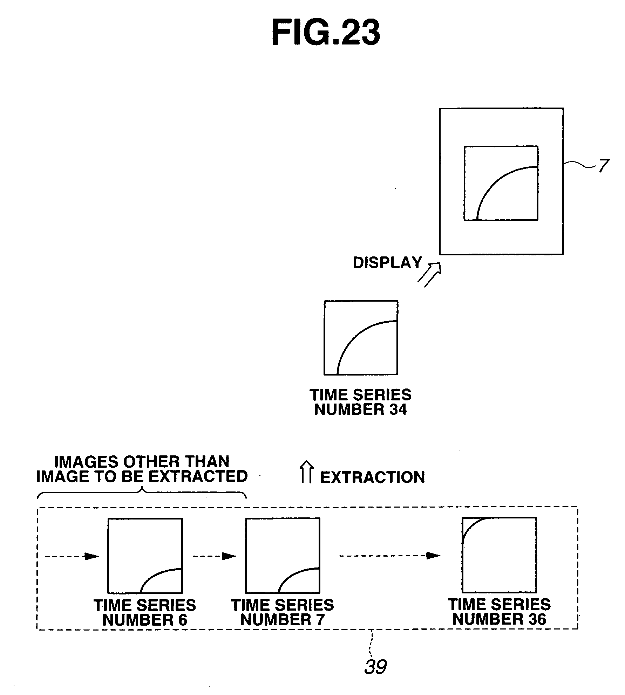

Figs. 1 to 23 are drawings relate to embodiments of the present invention. Fig. 1 is a view illustrating essential parts of an endoscope device according to an embodiment of the present invention. Fig. 2 is a view illustrating an internal configuration of the endoscope device according to the embodiment of the present invention. Fig. 3 is a view illustrating a configuration of a rotation filter provided in a light source section in the endoscope device according to the embodiment of the present invention. Fig. 4 is a view illustrating transmission characteristics of an RGB filter provided in the rotation filter shown in Fig. 3. Fig. 5 is a view illustrating transmission characteristics of a fluorescence observation filter provided in the rotation filter shown in Fig. 3. Fig. 6 is a view illustrating a configuration of a band switching filter provided in the light source section in the endoscope device according to the embodiment of the present invention. Fig. 7 is a view illustrating transmission characteristics of an ordinary/fluorescence observation filter and an infrared light observation filter provided in the band switching filter shown in Fig. 6. Fig. 8 is a view illustrating transmission characteristics of a NBI filter provided in the band switching filter shown in Fig. 6. Fig. 9 is a view illustrating transmission characteristics of an excitation light cut filter provided in an electronic endoscope in the endoscope device according to the embodiment of the present invention. Fig. 10 is a view illustrating an example of setting screens of a processor provided in the endoscope device according to the embodiment of the present invention. Fig. 11 is a view illustrating an example of configurations of an image capturing section provided in the electronic endoscope in the endoscope device according to the embodiment of the present invention. Fig. 12 is a view illustrating an example different from the example shown in Fig. 11 illustrating a configuration of the image capturing section provided in the electronic endoscope in the endoscope device according to the embodiment of the present invention. Fig. 13 is a flowchart illustrating an example of processing performed in the processor in a case that an observation mode is switched from an observation mode to another mode in the endoscope device according to the embodiment of the present invention. Fig. 14 is a view illustrating an example of writing and readout states of an image capture signal in a memory section in a case that the observation mode is switched from an observation mode to another mode in the endoscope device according to the embodiment of the present invention. Fig. 15 is a view illustrating an example different from the example shown in Fig. 10 illustrating a setting screen of the processor provided in the endoscope device according to the embodiment of the present invention. Fig. 16 is a flowchart illustrating an example different from the example shown in Fig. 13 illustrating processing performed in the processor in the case that the observation mode is switched from an observation mode to another mode in the endoscope device according to the embodiment of the present invention. Fig. 17 is a view illustrating an example of pre-freeze processing performed in the processor provided in the endoscope device according to the embodiment of the present invention. Fig. 18 is a view illustrating an example of writing and readout states of an image capture signal in a synchronization circuit in a case that the observation mode is switched from an observation mode to another mode in the endoscope device according to the embodiment of the present invention. Fig. 19 is a view illustrating an example different from the example shown in Fig. 18 illustrating writing and readout states of the image capture signal in the synchronization circuit in a case that the observation mode is switched from an observation mode to another mode in the endoscope device according to the embodiment of the present invention. Fig. 20 is a view illustrating an example different from the examples shown in Figs. 18 and 19 illustrating writing and readout states of the image capture signal in the synchronization circuit in the case that the observation mode is switched from an observation mode to another mode in the endoscope device according to the embodiment of the present invention. Fig. 21 is a view illustrating an example different from the example shown in Fig. 14 illustrating writing and readout states of the image capture signal in the memory section in the case that the observation mode is switched from an observation mode to another mode in the endoscope device according to the embodiment of the present invention. Fig. 22 is a schematic view illustrating another example of the pre-freeze processing performed in the processor provided in the endoscope device according to the embodiment of the present invention. Fig. 23 is a schematic view illustrating processing to be performed concomitantly with the processing shown in Fig. 22 in the processor provided in the endoscope device according to the embodiment of the present invention.

As shown in Fig. 1, an endoscope device 1 that functions as an image processing device essentially includes an electronic endoscope 2 for capturing an image of a subject, a light source section 3 that functions as light source means for supplying illumination light to the electronic endoscope 2, a processor 6, a monitor 7 for displaying an image of a subject based on an image signal outputted from the processor 6, a monitor image photographing device 8A for photographing an image (hereinafter, also referred to as an endoscopic image) of a subject displayed on the monitor 7 that functions as display means, an image filing device 8B that is connected to the processor 6 to record image information or the like, and a keyboard 9 for outputting an instruction signal for instructing the processor 6 to process an image and inputting patient's data or the like.

The processor 6 includes a video processing block 4 for processing the image capture signal outputted from the electronic endoscope 2, an image processing block 5 for performing image processing with respect to the signal outputted from the video processing block 4 and outputting an image signal, and an image recording section (not shown) for recording the image signal outputted from the image processing block 5.

The elongated electronic endoscope 2 includes, for example, a movable insertion portion 11, a wide operation portion 12 is consecutively provided to a back end of the insertion portion 11, and, further, a flexible universal code 13 is extendedly provided from a side part of the back end side of the operation portion 12. A connector 14 provided at an end part of the universal code 13 is detachably connectable to a connector receiving section 15 of the processor 6.

In the insertion portion 11 of the electronic endoscope 2, a rigid tip part 16, a curvable curved section 17 adjacent to the tip part 16, and a flexible long flexible section 18 are sequentially provided from the tip side.

A curving operation knob 19 provided to the operation portion 12 of the electronic endoscope 2 can curve the curved section 17 in a horizontal direction or a vertical direction in response to a user's rotation operation. The operation portion 12 of the electronic endoscope 2 includes an insertion opening 20 (not shown) communicating with an operative instrument channel provided in the insertion portion 11.

At a top part of the operation portion 12 of the electronic endoscope 2, a scope switch 10 that includes switches such as a freeze switch functioning as freeze means for performing a freeze instruction, a release switch for performing a release instruction, and an observation mode selection switch for performing an observation mode selection instruction, is provided.

For example, in a case that a freeze instruction is issued by operating the scope switch 10, from the scope switch 10, an instruction signal is outputted. The instruction signal outputted from the scope switch 10 is inputted in a control circuit 40, which will be described below, provided in the processor 6. The control circuit 40, based on the instruction signal outputted from the scope switch 10, controls a memory section 39, which will be described below, so that a freeze image is displayed.

A scope ID memory 48 provided in the electronic endoscope 2, when the electronic endoscope 2 is connected with the processor 6, outputs information such as correction parameters about observation modes (ordinary observation, fluorescence observation, NBI, and infrared observation) processable in the electronic endoscope 2, parts (upper digestive tract, lower digestive tract, and bronchus) observable by the electronic endoscope 2, and difference in equipment (difference due to models and individual difference are included) of the electronic endoscope 2 or the like to the control circuit 40 and a CPU 56.

An identification information circuit 43 provided in the electronic endoscope 2, when the electronic endoscope 2 is connected with the processor 6, for example, outputs information such as model information to the control circuit 40 and the CPU 56.

A white balance adjustment circuit 38 provided in the video processing block 4 of the processor 6 processes a signal in the electronic endoscope 2, for example, a signal for correcting difference in color tones generated due to difference of models such as transmission characteristics in an optical system.

Now, a recording method of an endoscopic image displayed on the monitor 7 is described.

A user operates the keyboard 9 and a front panel 55 of the processor 6, or the like to output an instruction signal for performing a freeze instruction to the control circuit 40. The control circuit 40, based on the instruction signal, executes a control corresponding to the freeze instruction.

The user further operates the keyboard 9 and the front panel 55 of the processor 6, or the like to output an instruction signal for performing a release instruction. The CPU 56, based on the instruction signal, in a case that a freeze image is not displayed, outputs a control signal based on the release instruction to the monitor image photographing device 8A while controlling to display the freeze image through the control circuit 40. The monitor image photographing device 8A, based on the control signal outputted from the CPU 56, photographs an endoscopic image to be displayed on the monitor 7.

Now, an image processing method is described.

The user operates the keyboard 9 and the front panel 55 of the processor 6, or the like to output an instruction signal for performing an image processing instruction. The CPU 56, based on the instruction signal, controls an IHb calculation circuit 61 of an IHb processing block 44, an IHb average value calculation circuit 62, a luminance detection circuit 67, an invalid region detection circuit 68, or the like to perform an image processing corresponding to the image processing instruction. Then, the user, for example, may stop the image processing executed in each section of the IHb processing block 44 at a desired timing by operating the keyboard 9 and the front panel 55 of the processor 6, or the like.

The user operates the scope switch 10 of the electronic endoscope 2 to output an instruction signal for performing an observation mode switching instruction. The control circuit 40, based on the instruction signal, controls a moving motor 31 and a motor 81, which will be described below, to move a rotation filter 27 and a band switching filter 80 so that the observation mode is switched from the ordinary observation mode to the fluorescence observation mode, for example.

Now, the electronic endoscope 2 and the light source section 3 will be described.

As shown in Fig. 2, the tip part 16 of the electronic endoscope 2 includes a lighting lens 21 and an image capturing section 30.

The image capturing section 30, as shown in Fig. 11, includes objective optical systems 22a and 22b for forming an image of a subject, a CCD 30a as image capturing means provided at the image-forming position of the objective optical system 22a for capturing the image of the subject formed with the objective optical system 22a, a CCD 30b as image capturing means provided at the image-forming position of the objective optical system 22b for capturing the image of the subject formed with the objective optical system 22b and capable of capturing a highly-sensitive as compared with the CCD 30a, a switching section 30c for switching drive states of the CCD 30a and CCD 30b based on a switching signal outputted from the control circuit 40, and an excitation light cut filter 32 disposed in front of the image-capturing face of the CCD 30b. The excitation light cut filter 32 has a function to shut out excitation light of 390 to 450 nm and extract fluorescence.

In the embodiment, the switching section 30c, in a case that the observation mode of the endoscope device 1 is switched to the ordinary observation mode, drives the CCD 30a, and in a case that the observation mode of the endoscope device 1 is switched to the fluorescence observation mode, drives the CCD 30b.

At a back end of the lighting lens 21, an output end that is an end of a light guide 23 made of a fiber bundle is disposed. The light guide 23 is provided so as to be inserted into the insertion portion 11, the operation portion 12, and the universal code 13, and an incident end that is another end is disposed in the connector 14. With the configuration of the light guide 23, the illumination light outputted from the light source section 3 in the processor 6 is, in a case that the connector 14 is connected with the processor 6, after being entered into the incident end of the light guide 23, outputted from the output end disposed at the back end side of the lighting lens 21 and irradiates the subject.

The light source 3 includes a lamp 24 having, for example, a xenon lamp for outputting illumination light including visible light. The illumination light outputted from the lamp 24 is entered into the rotation filter 27 that is driven by a motor 26 through an aperture 25 arranged on an optical path of the lamp 24. Then, the illumination light transmitted and outputted from the rotation filter 27 is converged by a condenser lens, and enters into the incident end of the light guide 23. The aperture 25 is driven in response to a drive state of an aperture motor 25a that is controlled by the controller 40.

In the rotation filter 27, as shown in Fig. 3, an RGB filter 28 for the ordinary observation is disposed at an inner circumference side of a concentric ring and a fluorescence observation filer 29 is disposed at an outer circumference side of the concentric ring. The rotation filter 27 is moved in a direction orthogonal to the optical path of the lamp 24 that is the direction indicated by the allow P in Fig. 2 by the moving motor 31 with the motor 26 for rotating the rotation filter 27. That is, in a case that the instruction to switch the observation mode is issued, the moving motor 31 moves the motor 26 and the rotation filter 27 so that the filter disposed on the optical path of the lamp 24 is switched. In the embodiment, in a case that the ordinary observation mode, the NBI mode, or the infrared observation mode is selected as the observation mode, the control circuit 40 outputs a switching signal for disposing the RGB filter 28 on the optical path of the lamp 24 to the moving motor 31. In a case that the fluorescence observation mode is selected as the observation mode, the control circuit 40 outputs a switching signal for disposing the fluorescence observation filter 29 on the optical path of the lamp 24 to the moving motor 31.

The RGB filter 28 includes an R filter 28a, a G filter 28b, and a B filter 28c that have transmission characteristics shown in Fig. 4 respectively. Specifically, the R filter 28a transmits a red waveband of 600 nm to 700 nm, the G filter 28b transmits a green waveband of 500 nm to 600 nm, and the B filter 28c transmits a blue waveband of 400 nm to 500 nm. In addition to the above-described configuration, for the infrared observation, the R filter 28a and the G filter 28b include a configuration to transmit a waveband of 790 to 820 nm. In addition to the above-described configuration, for the infrared observation, the B filter 28c includes a configuration to transmit a waveband of 900 to 980 nm. Accordingly, the processor 6, in the ordinary observation mode, for example, synthesizes a image capture signal created based on the image of the subject captured under the illumination light transmitted the R filter 28a, a image capture signal created based on the image of the subject captured under the illumination light transmitted the G filter 28b, and a image capture signal created based on the image of the subject captured under the illumination light transmitted the B filter 28c so as to form an observation image as an image of the subject for the ordinary observation that is an image of the subject substantially similar to that observed with the naked eye.

The fluorescence observation filter 29 includes a G2 filter 29a, an E filter 29b, and a R2 filter 29c that have transmission characteristics shown in Fig. 5 respectively. Specifically, the G2 filter 29a transmits a waveband of 540 nm to 560 nm, the E filter 29b transmits a waveband of 400 nm to 470 nm, and the R2 filter 29c transmits a waveband of 600 nm to 620 nm. As shown in Fig. 5, the transmittances of the G2 filter 29a and the R2 filter 29c are set to be lower than that of the E filter 29b. Accordingly, the processor 6, in the fluorescence observation mode, for example, synthesizes a image capture signal created based on the image of the subject captured under the illumination light transmitted the G2 filter 29a (hereinafter, referred to as a G2 signal), a image capture signal created based on the image of the subject captured under the illumination light transmitted the R2 filter 29c (hereinafter, referred to as a R2 signal), and a fluorescence signal that is an image capture signal created based on the image of fluorescence generated by the subject so as to form an observation image as an image of the subject for the fluorescence observation that is an image of a pseudo color image of the image of fluorescence generated by the subject.

A band switching filter 80 includes, as shown in Fig. 6, an ordinary/fluorescence observation filter 80a, a NBI filter 80b, and an infrared observation filter 80c. The ordinary/fluorescence observation filter 80a and the infrared observation filter 80c have the transmission characteristics shown in Fig. 7. The NBI filter 80b, as shown in Fig. 8, has a trimodal filter that transmits three discrete bands with one filter.

In the excitation light cut filter 32 in the electronic endoscope 2, the transmission band has the transmission characteristics shown in Fig. 9 that is different from that of the E filter 29b shown in Fig. 4.

The band switching filter 80 is driven to rotate with the motor 81 in response to a filter switching instruction signal issued by the CPU 56. Then, in the band switching filter 80, with the rotation drive of the motor 81, in a case that the ordinary observation and the fluorescence observation is performed, the ordinary/fluorescence observation filter 80a is disposed on the optical path of the lamp 24, in a case that the NBI is performed, the NBI filter 80b is disposed on the optical path of the lamp 24, and in a case that the infrared observation is performed, the infrared observation filter 80c is disposed on the optical path of the lamp 24.

With a combination of the rotation filter 27 and the band switching filter 80 disposed on the optical path of the lamp 24, in a case that the ordinary observation is performed, light that has the red, green, and blue bands is sequentially outputted from the light source section 3. In a case that the NBI is performed, with a combination of the transmission characteristics shown in Fig. 4 and the transmission characteristics shown in Fig. 8, light that has a band of 600 nm to 630 nm, a band of 530 nm to 660 nm, and a band of 400 nm to 430 nm is sequentially outputted from the light source section 3. In a case that the infrared observation is performed, with a combination of the transmission characteristics shown in Fig. 4 and the transmission characteristics shown in Fig. 7, light that has a band of 790 nm to 820 nm, a band of 790 nm to 820 nm, and a band of 900 nm to 980 nm is sequentially outputted from the light source section 3. In a case that the fluorescence observation is performed, with a combination of the transmission characteristics shown in Fig. 5 and the transmission characteristics shown in Fig. 7, light that has a band of 540 nm to 560 nm, a band of 390 nm to 450 nm, and a band of 600 nm to 620 nm is sequentially outputted from the light source section 3. The light that has the band of 390 nm to 450 nm is excitation light for exciting self-fluorescence from an organism.

The illumination light entered into the light guide 23 of the electronic endoscope 2 is irradiated to a subject such as a living tissue from the tip part 16 of the electronic endoscope 2. The light scattered, reflected, and emitted in the subject is formed as an image and the image is captured in the image capturing section 30 provided in the tip part 16 of the electronic endoscope 2.

The illumination light entered into the light guide 23 of the electronic endoscope 2 is introduced in the tip part 16 with the light guide 23, transmits the lighting lens 21 installed in an irradiation window at the tip surface, and irradiates the subject. In such a case, in the ordinary observation mode, the light becomes surface sequential illumination light of R (red), G (green), and B (blue). In the fluorescence observation mode, the light becomes surface sequential illumination light of G2, E, and R2.

The CCDs 30a and 30b are driven synchronized with the rotation of the rotation filter 27 when a CCD drive signal is applied by a CCD driver 33 respectively. The CCDs 30a and 30b perform photoelectric conversion with respect to the image formed with the objective optical systems 22a and 22b respectively and outputs as image capture signals. Then, to the processor 6, the image capture signals corresponding to the irradiation light transmitted the RGB filter 28 and the fluorescence observation filter 29 provided in the rotation filter 27 are outputted respectively.

The control circuit 40 or the CPU 56 may operate an electronic shutter for variably controlling charge storage time with the CCDs 30a and 30b by controlling the CCD driver 33.

Now, a description will be made with respect to the processor 6.

The time series image capture signals outputted form the CCDs 30a and 30b are inputted in an amplifier 34 provided in the video processing block 4, and, converted into signals of a certain signal level, for example, from 0 to 1 volt.

In such a case, in the ordinary observation mode, the time series image capture signals become color signals of R, G, and B respectively. In the fluorescence observation mode, the time series image capture signals become signals of G2, fluorescence, and R2. In the NBI mode and infrared observation mode, the time series image capture signals become signals corresponding to each illumination light.

The image capture signals outputted from the amplifier 34 are converted into digital signals in an A/D converter 35 and outputted to an automatic gain control circuit (hereinafter, referred to as an AGC circuit) 36. The image capture signals outputted from the A/D converter 35 are automatically controlled to be appropriate signal levels by controlling the gains in the AGC circuit 36 and outputted.

The image capture signals outputted from the AGC circuit 36 is inputted into a selector 37 of one input and three outputs. Then, in the image capture signals time sequentially sent, in the selector 37, the each of the color signals of R, G, and B or the G2 signal, the fluorescence signal, and the R2 signal are switched respectively and inputted into the white balance adjustment circuit 38 in order. The white balance adjustment circuit 38, in a case that a white subject to be a reference is captured, controls a gain, that is, white balance, such that signal levels of each of the color signals of R, G, and B are equal. The image capture signals outputted from the white balance adjustment circuit 38 are inputted into a memory section 39 that is a part of freeze image generation means and functions as storage means. Then, the white balance adjustment may be automatically performed by reading an adjustment value for the white balance from the scope ID memory 48 provided in the electronic endoscope conduit 2.

The image capture signals of the each of the color signals of R, G, and B time sequentially inputted are stored on an R memory 39r, a G memory 39g, and a B memory 39b that are included in the memory section 39 and function as freeze memories respectively.

With the configuration of the memory section 39, in the ordinary observation mode, the R color signal is stored on the R memory 39r, the G color signal is stored on the G memory 39g, and the B color signal is stored on the B memory 39g respectively. In the fluorescence observation mode, the G2 signal is stored on the R memory 39r, the fluorescence signal is stored on the G memory 39g, and the R2 signal is stored on the B memory 39b respectively.

The control circuit 40 controls the A/D conversion with the A/D converter 35, the switching of the selector 37, the control at the time of the white balance adjustment, and writing and reading of the image capture signals such as the each of the color signals of R, G, and B with respect to the R memory 39r, the G, memory 39g, and the B memory 39b in the memory section 39. That is, the image capture signals outputted from the white balance adjustment circuit 38 are written on the memory section 39 based on the writing signals outputted from the control circuit 40 to the memory section 39. The image capture signals written on the memory section 39 are read out from the memory section 39 based on the reading signals outputted from the control circuit 40 to the memory section 39.

The control circuit 40 sends a reference signal to a synchronization signal generation circuit (in Fig. 2, expressed as SSG) 41, and the synchronization signal generation circuit 41 generates a synchronization signal synchronized with the signal. In a case that the control circuit 40 executes a control to forbid writing on the R memory 39r, the G memory 39g, and the B memory 39b, a still image is displayed on the monitor 7. The control to forbid writing on the R memory 39r, the G memory 39g, and the B memory 39b may be performed in a synchronization circuit 53.

The image capture signals outputted from the A/D converter 35 are photometrically measured in a photometric circuit 42 and inputted into the control circuit 40.

The control circuit 40 compares an average value obtained by performing integration to the signal photometrically measured in the photometric circuit 42 with a reference value of the case of appropriate brightness. Then, the control circuit 40 outputs a photochromic signal according to the comparison result to drive the aperture motor 25a. Further, the control circuit 40 controls an opening amount of the aperture 25 that is driven synchronized with the aperture motor 25a to adjust quantity of the illumination light outputted from the light source 3 so that the difference between the average value and the reference value becomes small.

To the aperture motor 25a, for example, a rotary encoder (not shown) is mounted to detect an aperture position corresponding to the opening amount of the aperture 25, and a detection signal of the rotary encoder is inputted into the control circuit 40. With the detection signal outputted from the rotary encoder, the control circuit 40 may detect the position of the aperture 25. The control circuit 40 is connected to the CPU 56. Accordingly, the CPU 56 can recognize the position of the aperture 25 detected in the control circuit 40.

Now, image processing available in the ordinary observation mode will be described.

In the ordinary observation mode, each of the color signals of R, G, and B read from the R memory 39r, the G memory 39g, and the B memory 39b is inputted into the IHb processing block 44 that is included in the image processing block 5 and performs processing such as a calculation of a value (hereinafter, referred to as IHb) correlating with an amount of hemoglobin as an amount of pigment to be blood information.

In the embodiment, the IHb processing block 44, for example, includes an IHb processing circuit section 45 for calculating an IHb value in each pixel in an interest region set in the setting screen of the processor 6 shown in Fig. 10, and performing pseudo image generation processing for displaying an IHb image displayed based on the IHb value as a pseudo color image, and an invalid region detection section 46 for detecting an invalid region not suitable for image processing with respect to the set interest region. Specifically, an IHb calculation circuit 61 performs an operation based on the following expression (1) to calculate values of the IHb in each pixel.

$$IHb = 32 \times \log_2 (R/G) \ldots \text{expression (1)}$$

In the expression (1), R denotes, in the interest region, data of an R image in a region other than the invalid region, and G denotes, in the interest region, data of a G image in the region other than the invalid region.

The signal outputted from the IHb processing block 44 is $\gamma$ corrected in a $\gamma$ correction circuit 50 and outputted. Further, in a post image processing circuit 51, a structure emphasis is performed and outputted. On the signal outputted from the post image processing circuit 51, in a character superposition circuit 52, data about a patient having the living tissue to be the subject and the average value of the IHb calculated in the IHb processing block 44 are superposed and then synchronized in the synchronization circuit 53. The synchronization circuit 53 includes three frame memories (not shown) inside the circuit, outputs synchronized signals such as RGB signals by simultaneously reading surface sequence signals after the surface sequence signal data is sequentially written on the frame memories.

The synchronized signals synchronized in the synchronization circuit 53 is inputted into three D/A converters in the D/A conversion section 54 respectively, converted into analog RGB signals or the like, and outputted to the monitor 7, the monitor image photographing device 8A, and the image filing device 8B respectively.

The processor 6, other than the above-described character superposition circuit 52, the synchronization circuit 53, and the D/A conversion section 54, includes a character superposition circuit 52a that has a substantially similar configuration to the character superposition circuit 52, a synchronization circuit 53a that has a substantially similar configuration to the synchronization circuit 53, and a D/A conversion section 54a that has a substantially similar configuration to the D/A conversion section 54.

An index image generation section 51a performs processing based on the signal outputted from the post image processing circuit 51, and outputs the processed signal to the character superposition circuit 52.

A detection circuit 57 performs processing based on the signals outputted from the image capturing section 30 and the identification information circuit 43, and outputs the processed signals to an interest region setting circuit 63.

The interest region setting circuit 63 performs processing based on the signals outputted from the CPU 56 and the detection circuit 57, and outputs the processed signals to the $\gamma$ correction circuit 50, the post image processing circuit 51, the IHb calculation circuit 61, an IHb average value calculation circuit 62, and an image synthesis/color matrix circuit 65.

A pseudo image generation circuit 64 performs processing based on the signals outputted from the CPU 56, the IHb calculation circuit 61, and an invalid region display circuit 69, and the processed signals are outputted to the image synthesis/color matrix circuit 65.

The invalid region display circuit 69 performs processing based on the signals outputted from the CPU 56 and an invalid region detection circuit 68, and the processed signals are outputted to the pseudo image generation circuit 64.

A speaker 70 notifies, for example, a state of the processor 6 by playing a predetermined sound based on the control by the CPU 56.

The control circuit 40 controls the writing and readout of the frame memories in the synchronization circuit 53

and the D/A conversion in the D/A conversion section 54. The CPU 56 controls the operation of the γ correction circuit 50, the post image processing circuit 51, and the character superposition circuit 52.

The monitor image photographing device 8A includes a monitor (not shown) for displaying a image or the like, the monitor has a substantially similar configuration to the monitor 7, and a photographing device (not shown), for example, a camera, for recording an image by photographing an image displayed on the monitor.

The user may display the image of the subject captured in the ordinary observation mode or output an instruction signal for instructing an IHb image on the monitor 7 or the like to the CPU 56 by operating a switch (not shown) provided in a front panel 55 of the processor 6 or the keyboard 9. The CPU 56 controls the IHb processing block 44 or the like based on the instruction signal outputted by operating a switch (not shown) provided in the front panel 55 of the processor 6 or the keyboard 9.

Now, image processing available in the each observation mode other than the ordinary observation mode will be described.

In a case that each section in the endoscope device 1 is set in the fluorescence observation mode, the CCD 30b is driven and the CCD 30a is stopped to drive. Accordingly, in the fluorescence observation mode, the CCD 30b may capture a self-fluorescent image generated by the subject. Further, at a timing at which substantially similar to the timing at which an observation mode other than the fluorescence observation mode is switched to the fluorescence observation mode, the light source section 3 sets the rotation speed of the rotation filter 27 to half of that in the one observation mode. Thus, the CCD 30b may capture the self- fluorescent image generated by the subject with a longer exposure time than that in the one observation mode other than the fluorescence observation mode, and output the captured self- fluorescent image as an image capture signal.

In the fluorescence observation mode, the each of the color signals of R, G, and B written on the R memory 39r, the G memory 39g, and the B memory 39b respectively is, in synchronization with the exposure time in the fluorescence observation mode, for example, a same signal read twice from each of the R memory 39r, the G memory 39g, and the B memory 39b respectively.

The read G2 signal, the fluorescence signal, and the R2 signal are outputted to the post image processing circuit 51 through the image synthesis/color matrix circuit 65 and a surface sequence circuit 66 or the like. Then, the post image processing circuit 51, using a color matrix, for example, processes the signals such that the G2 signal is displayed in red color, the fluorescence signal is displayed in green color, and the R2 signal that the signal level is reduced to half is displayed in blue color on the monitor 7 as a pseudo color display.

In a case that the each section in the endoscope device 1 is set in the NBI mode or the infrared observation mode, the CCD 30a is driven and the CCD 30b is stopped to drive. In the case that the each section in the endoscope device 1 is set in the NBI mode or the infrared observation mode, an exposure is performed for substantially similar exposure time to that in the ordinary observation mode. Accordingly, the CCD 30a captures an image of a subject in substantially similar exposure time to that in the ordinary observation mode and outputs the image of the subject as an image capture signal. Further, in the case that the each section in the endoscope device 1 is set in the NBI mode or the infrared observation mode, the image of the subject is color displayed on the monitor 7 with each color signal and color matrix.

Now, in a case that an observation mode in the endoscope device 1 is switched from one observation mode to another observation mode will be described.

For example, in a case that the one observation mode is the ordinary observation mode and the other observation mode is the fluorescence observation mode will be described.

Before a process shown in step S 1 of Fig. 13 is performed, the control circuit 40 had outputted a writing signal to the memory section 39. In the state that the outputted writing signal is inputted from the control circuit 40, the memory section 39 may write an image capture signal.

In the processing shown in step S1 of Fig. 13, in a case that the control circuit 40 detects the ordinary observation mode is changed to the fluorescence observation mode, at step S2 in Fig. 13, the control circuit 40 controls to create a still image and outputs the image by outputting a switching signal to the synchronization circuit 53.

Then, at step S3 in Fig. 13, the control circuit 40 outputs the switching signal to the switching section 30c to drive the CCD 30b as one CCD and stop the drive of the CCD 30a as another CCD. In response to the switching signal outputted from the control circuit 40, the switching section 30c switches the drive states of the CCDs 30a and 30b. Further, the control circuit 40 executes the above-described processing shown in step S3 of Fig. 13 and stops the output of the writing signal to the memory section 39. In response to the instruction, the memory section 39 stops the writing of the image capture signal at the timing the input of the writing signal outputted from the control circuit 40 is stopped. Then, at step S4 in Fig. 13, the control circuit 40 changes a rotation speed of the rotation filter 27, for example, changes the rotation speed to half in the ordinary observation mode.

At steps S5 and S6 in Fig. 13, the control circuit 40 counts a predetermined time period. In a case that the ordinary observation mode is switched to the fluorescence observation mode, the predetermined time period is, for example, three seconds.

In a case the control circuit 40 detects the predetermined time period has passed, resumes the output of the writing signal to the memory section 39, and at step S7 in Fig. 13, controls to stop the output of the still image by outputting a switching completion signal to the synchronization circuit 53. In response to the signal, the memory section 39 releases the stop of writing of the image capture signal at the timing the input of the writing signal outputted from the control circuit 40 is resumed.

The control circuit 40, in the predetermined time period, may set an inoperative time to invalidate each instruction about operation of the image to be performed in any of the keyboard 9, the scope switch 10, and the front panel 55 of the processor 6.

Specifically, the control circuit 40 having functions of image operation invalidation means and image operation invalidation release means may invalidate each instruction such as a freeze instruction, a release instruction, an image emphasis instruction, a color conversion instruction, an enlarged display instruction, an observation mode switching instruction, and a comment input instruction to be performed in any of the keyboard 9, the scope switch 10, and the front panel 55 of the processor 6 that has a function as image operation means for the inoperative time in the predetermined time period. In a case that the endoscope device 1 has an air feeding function, with respect to an air feeding instruction performed in the scope switch 10 or the like, the control circuit 40 may not set the inoperative time. The above-described setting of the inoperative time may not be performed in the control circuit 40, but may be performed, for example, in the CPU 56.

Then, at step S8 shown in Fig. 13, the control circuit 40 instructs the synchronization circuit 53 to resume the output of the moving image and instructs the post image processing circuit 51 as display image size changing means to perform a processing appropriate for outputting the moving image, for example, a processing to change the size of an image displayed on the monitor 7 or a processing to adjust the masking size.

In the processing to change the image size performed in the post image processing circuit 51, for example, by changing the "fluorescence observation display size" on the setting screen of the processor 6 shown in Fig. 10, the image size displayed on the monitor 7 may be set to be a desired size.

Now, processing for creating a still image and switching a moving image to be executed in the synchronization circuit 53 will be described.

In a case of time series numbers 1 to 4 shown in Fig. 18, that is, in a case of the ordinary observation mode, the synchronization circuit 53 sequentially writes image capture signals that have each color signal of R, G, and B on three frame memories (not shown) provided inside, and simultaneously read the written image capture signals, and then, outputs synchronized RGB signals.

For example, at a time the processing shown in step S2 of Fig. 13 is executed, in a case that the switching signal outputted from the control circuit 40 is inputted at a timing of the time series number 4 shown in Fig. 18, that is, the ordinary observation mode is switched to the fluorescence observation mode, at the timing of the time series number 4 shown in Fig. 18, the synchronization circuit 53 stops the writing of the image capture signals on the three frame memories (not shown), creates a still image and outputs the image.

The control circuit 40, at the timing of the time series number 4 shown in Fig. 18, in a case that the switching signal is outputted to the synchronization circuit 53, for example, at a timing of the time series number 5 shown in Fig. 18, starts processing after step S3 in Fig. 13. The synchronization circuit 53, in response to the above-described operation of the control circuit 40, for example, from the time series number 5 to the time series number 10 shown in Fig. 18, that is, before the switching completion signal is outputted from the control circuit 40, continues to stop the writing of the image capture signals onto the three frame memories (not shown) and continues to output the still image created at the timing of the time series number 4 shown in Fig. 18.

Then, at a timing of the time series number 11 shown in Fig. 18, in a case that the switching completion signal is outputted to the synchronization circuit 53, the control circuit 40, for example, at a timing of the time series number 11 shown in Fig. 18, starts processing after step S7 in Fig. 13. The synchronization circuit 53, in response to the switching completion signal outputted from the control circuit 40, at the timing of the time series number 11 shown in Fig. 18, that is, at the timing the switching completion signal inputted from the control circuit 40 is inputted, releases the stop of writing of the image capture signals onto the three frame memories (not shown), and stops the output of the still image created at the timing of the time series number 4 shown in Fig. 18. The synchronization circuit 53 sequentially writes the image capture signals that include the G2 signal, the fluorescence signal, and the R2 signal onto the three frame memories (not shown) provided inside of the circuit as synchronization memories, simultaneously reads the written image capture signals, and outputs the synchronized signals. Thus, the self-fluorescent image is displayed as a moving image on the monitor 7.

It is to be understood that that the synchronization circuit 53 is not limited to release the stop of the writing of the image capture signals onto the three frame memories (not shown) at the timing the switching completion signal is inputted from the control circuit 40. The synchronization circuit 53 may release the stop of the writing of the image capture signals onto the three frame memories (not shown), for example, at certain timing appropriate for the observation mode such as the fluorescence observation after the switching completion signal is inputted from the control circuit 40.

As described above, at the time the one observation mode is switched to the other observation mode, the processing to display the still image on the monitor 7 is performed. Accordingly, for example, noise generated at the time the one CCD in the image capturing section 30 is switched to the other CCD, color change generated while the rotation speed of the rotation filter 27 is changed to a predetermined rotation speed, and color change generated until the switch of the band switching filter 80 is completed may be prevented. As a result, the processor according to the embodiment may output the still image suitable for recording while the one observation mode is switched to the other observation mode.

In a case that the one observation mode is the fluorescence observation mode and the other observation mode is the ordinary observation mode, in the processing shown at step S3 in Fig. 13, the control circuit 40 instructs the switching section 30c of the image capturing section 30 to drive the CCD 30a as the one CCD and stop the drive of the CCD 30b as the other CCD. Further, in a case that the fluorescence observation mode is switched to the ordinary observation mode, in the processing shown at step S4 in Fig. 13, the control circuit 40, for example, doubles the rotation speed of the rotation filter 27, and in the processing shown at steps S5 and S6 in Fig. 13, as the predetermined time period, counts every 1.5 seconds.

The synchronization circuit 53 that is a part of the freeze image generation means and functions as the storage means, to display the image on the monitor 7, includes a configuration to generate images of an odd field and an even field and output the images. Then, the still image outputted from the synchronization circuit 53 at the processing shown in step S2 of Fig. 13 may be outputted in a state that the images of the odd field and even field are shifted. In such a case, for example, the synchronization circuit 53, before the processing shown in step S2 of Fig. 13 is executed, instructs the memory section 39 to perform processing to create still images in advance. Then, still images of lower shift may be generated and outputted. The still images created in the memory section 39 with the above-described processing performed by the synchronization circuit 53 may be the image of the time an ordinary freeze instruction is issued or may be the image of the time just before the observation mode is switched to the fluorescence observation mode.

Further, the still image outputted from the synchronization circuit 53 at the processing shown in step S2 of Fig. 13 may be the image in the odd field applied to the image of the even field.

The above-described processing shown in Fig. 13 may be applied not only to the case that the electronic endoscope 2 includes the image capturing section 30 having the two CCDs shown in Fig. 11, but may be applied to a case that, as shown in Fig. 12, the electronic endoscope 2 includes an image capturing section 30A having one CCD.

The image capturing section 30A, as shown in Fig. 12, includes an objective optical system 22c for forming an image of a subject, a CCD 30d as image capturing means provided at the image-forming position of the objective optical system 22c for capturing the image of the subject formed with the objective optical system 22c, and the excitation light cut filter 32 disposed in front of the image-capturing face of the CCD 30d. In a case that the electronic endoscope 2 includes the image capturing section 30A, the control circuit 40 does not execute the processing shown in step S3 of Fig. 13. Further, in the case that the electronic endoscope 2 includes the image capturing section 30A, in the processing shown in step S8 of Fig. 13, the control circuit 40 instructs the synchronization circuit 53 to resume the output of the moving image without performing the adjustment of the image size and masking size.

Now, processing performed by the processor 6 in a case that right after an observation mode in the endoscope device 1 is switched from one mode to another mode, a freeze instruction is issued in the scope switch 10 or the like will be described.

On the memory section 39, in synchronize with the rotation speed of the rotation filter 27, image capture signals outputted from the image capturing section 30 are time-sequentially written. In the case that right after the observation mode in the endoscope device 1 is switched from the one mode to the other mode, the freeze instruction is issued in the scope switch 10 or the like, a color shift detection circuit 47 detects a least color shifted image capture signal out of the image capture signals written on the memory section 39, and performs processing to display a still image according to the image capture signal on the monitor 7 as a freeze image, that is, pre-freeze processing.

Specifically, for example, as shown in Fig. 14, in a case that the freeze instruction is issued at a timing of F2, that is, at a timing of the time series number 21, the color shift detection circuit 47 detects a least color shifted image capture signal out of the image capture signals written on the memory section 39 at the time between the time series number 13 and the time series number 20, and performs the pre-freeze processing to display the still image according to the image capture signal on the monitor 7 as the freeze image.

Further, as shown in Fig. 14, in a case that the freeze instruction is issued at a timing of F1, that is, a timing of the time series number 12, right after the observation mode in the endoscope device 1 is switched from the one mode to the other mode, the color shift detection circuit 47 invalidates the freeze instruction and does not execute the pre-freeze processing. Specifically, the color shift detection circuit 47, in Fig. 14, even if the freeze instruction is issued at a timing between the time series number 5 and the timer series number 18, invalidates the freeze instruction and does not execute the pre-freeze processing for displaying the freeze image on the monitor 7.

With the above-described processing being performed by the color shift detection circuit 47 that is a part of the freeze image generation means, for example, it is prevented that either of the still image according to the image capture signal written in the memory section 39 at a timing between the time series number 5 and the time series number 10 shown in Fig. 14 by Δ, at which the possibility of existence of noise is high, or, the still image according to the image capture signal written in the memory section 39 at a timing 4 at which the switch of the CCD in the image capturing section 30 has not completed is displayed on the monitor 7 as the freeze image. As a result, the processor 6 according to the embodiment, in the case that the freeze instruction is issued right after the one observation mode is switched to the other observation mode, may prevent the image not suitable for recording of still images from being outputted by invalidating the freeze instruction.

The color shift detection circuit 47 is not limited to determine the time period for invalidating the freeze instruction by the time series numbers, but may decide, for example, by the predetermined time.

Specifically, in a case that the color shift detection circuit 47, in the processing shown in step S11 of Fig. 16, detects that the one observation mode is switched to the other observation mode through the control circuit 40, at the processing shown in step S12 of Fig. 16, determines whether the exposure time is changed. That is, in the processing shown in step S12 of Fig. 16, in a case that the color shift detection circuit 47 detects that the observation mode in the endoscope device 1 is switched from the ordinary observation mode to the fluorescence observation mode, or, from the fluorescence observation mode to the ordinary observation mode, determines that the exposure time is changed.

Then, in the processing shown in step S 13 of Fig. 16, in the case that the color shift detection circuit 47 detects that the exposure time is changed, set the time period for invalidating the freeze instruction to 3 seconds. Further, in the processing shown in step S14 of Fig. 16, in a case that the color shift detection circuit 47 detects that the exposure time is not changed, set the time period for invalidating the freeze instruction to 0.1 seconds.

In the processing shown in step S 15 of Fig. 16, the color shift detection circuit 47 invalidates the freeze instruction and in the processing shown in step S 16 of Fig. 16, starts to count the time passed since the one observation mode is switched to the other observation mode.

Then, in the processing shown in step S 17 of Fig. 16, in a case that the color shift detection circuit 47 detects that the time period for invalidating the freeze instruction has passed, in the processing shown in step S18 of Fig. 16, the freeze instruction is validated.

In the pre-freeze processing performed in the color shift detection circuit 47, for example, a processing level value may be set for the setting values 1 to 7 shown as "freeze level" on the setting screen of the processor 6 shown in Fig. 15.

For example, in a case that the processing level value is set to 1 and the freeze operation is executed at the timing of F2 shown in Fig. 14, the color shift detection circuit 47 detects a least color shifted image capture signal from the image capture signals written on the memory section 39 between the time series number 16 and the time series number 20 and executes the pre-freeze processing such that the still image according to the image capture signal is displayed on the monitor 7 as the freeze image.

Further, for example, in a case that the processing level value is set to 2 and the freeze operation is executed at the timing of F2 shown in Fig. 14, the color shift detection circuit 47 detects a least color shifted image capture signal from the image capture signals written on the memory section 39 between the time series number 13 and the time series number 20 and executes the pre-freeze processing such that the still image according to the image capture signal is displayed on the monitor 7 as the freeze image.

Further, in a case that the processing level value is set to 3 and the freeze operation is executed at the timing of F2 shown in Fig. 14, the color shift detection circuit 47 detects a least color shifted image capture signal from the image capture signals written on the memory section 39 between the time series number 10 and the time series number 20 and executes the pre-freeze processing such that the still image according to the image capture signal is displayed on the monitor 7 as the freeze image.

As described above, the color shift detection circuit 47 performs the pre-freeze processing depending on the set processing level value, by increasing or reducing the time period at which the image capture signal to be processed is written from the image capture signals written on the memory section 39. Then, the color shift detection circuit 47 may perform processing to increase or reduce the time period for invalidating the freeze instruction depending on the set processing level value described above.

Further, the color shift detection circuit 47, for example, may set the time period for invalidating the freeze instruction in advance as a certain period during and right after the one observation mode is switched to the other observation mode, for example, the time period between the time series number 5 and the time series number 14 shown in Fig. 14, and at the timing the freeze instruction is issued, determines the processing level of the pre-freeze processing.

Specifically, the color shift detection circuit 47, in the processing shown in step S21 of Fig. 17, stores a first processing level in the pre-freeze processing set by the operator or the like. Then, the color shift detection circuit 47, in the processing shown in step S22 of Fig. 17, as a temporary initial value of the pre-freeze level, sets a second processing level value, and, as a time period for invalidating the freeze instruction, sets a certain period during and right after the one observation mode is switched to the other observation mode. Then, in the processing shown in step S23 of Fig. 17, in a case that the color shift detection circuit 47 detects that the one observation mode is switched to the other observation mode through the control circuit 40, in the processing shown in step S24 of Fig. 17, count of the time passed since the one observation mode is switched to the other observation mode is started. Further, the color shift detection circuit 47, in the processing shown in step S25 of Fig. 17, every time a predetermined time (for example, 0.1 second) has passed since the one observation mode is switched to the other observation mode, increases the second processing level value.

In the processing shown in step S26 of Fig. 17, in a case that the color shift detection circuit 47 detects that the freeze instruction is issued, in the processing shown in step S27 of Fig. 17, the color shift detection circuit 47 compares the first processing level value to the second processing level value at the timing the freeze instruction is issued. In a case that the color shift detection circuit 47 detects that the first processing level value is larger than the second processing level value, in the processing shown in step S28 of Fig. 17, executes a pre-freeze processing based on the first processing level value. In a case that the color shift detection circuit 47 detects that the first processing level value is smaller than the second processing level value, in the processing shown in step S29 of Fig. 17, executes a pre-freeze processing based on the second processing level value.

In the setting screen of the processor 6 shown in Fig. 15, for example, the set value shown as "observation mode switching time" denotes time for displaying a still image at a time of switching the observation mode. The user may set the still image display time in the observation mode switching to a desired time by changing the set value displayed on the setting screen of the processor 6 shown in Fig. 15, for example, using the keyboard 9 as observation mode switching time setting means. Then, the processor 6 performs the following processing in each section in response to the change of the set value by the user.

First, control to be performed by the control circuit 40, for example, in a case that the observation mode switching time is set to "2" will be described.

For example, at a timing of time series number 3 shown in Fig. 19, in a case that the control circuit 40 outputs a switching signal to the synchronization circuit 53, at a timing of time series number 4 shown in Fig. 19, the control circuit 40 starts the above-described processing after step S3 shown in Fig. 13. The synchronization circuit 53, in response to the above-described operation of the control circuit 40, for example, in the time period between the time series number 5 and the time series number 21 shown in Fig. 19, continues to stop the writing of the image capture signals on the three frame memories (not shown) and continues to output the still image created at the timing of the time series number 3 shown in Fig. 19.

Then, based on the set value of the observation mode switching time, for example, at a timing of time series number 22 shown in Fig. 19, the control circuit 40 outputs a switching completion signal to the synchronization circuit 53 and starts the processing after step S7 shown in Fig. 13. The synchronization circuit 53, based on the switching completion signal outputted from the control circuit 40, at the timing of time series number 22 shown in Fig. 19, that is, at the timing the switching completion signal from the control circuit 40 is inputted, releases the stop of the writing of the image capture signals on the three frame memories (not shown) and stops the output of the still image created at the timing of the time series number 3 shown in Fig. 19. Then, the synchronization circuit 53 sequentially writes the image capture signals including the G2 signal, the fluorescence signal, and the R2 signal on the three frame memories (not shown) provided in the circuit as synchronization memories, simultaneously reads the written image capture signals and outputs the synchronized image capture signals. Thus, a self-fluorescent image is displayed as a moving image.

Next, control to be performed by the control circuit 40, for example, in a case that the observation mode switching time is set to "1" as a smallest value will be described.

For example, at a timing of time series number 3 shown in Fig. 20, in a case that the control circuit 40 outputs a switching signal to the synchronization circuit 53, at a timing of time series number 4 shown in Fig. 19, the control circuit 40 starts the above-described processing after step S3 shown in Fig. 13. The synchronization circuit 53, in response to the above-described operation of the control circuit 40, for example, in the time period between the time series number 5 and the time series number 12 shown in Fig. 20, continues to stop the writing of the image capture signals on the three frame memories (not shown) and continues to output the still image created at the timing of the time series number 3 shown in Fig. 20.

Then, based on the set value of the observation mode switching time, for example, at a timing of time series number 13 shown in Fig. 20, the control circuit 40 outputs a switching completion signal to the synchronization circuit 53 and starts the processing after step S7 shown in Fig. 13. The synchronization circuit 53, based on the switching completion signal outputted from the control circuit 40, at the timing of time series number 13 shown in Fig. 20, that is, at the timing the switching completion signal from the control circuit 40 is inputted, releases the stop of the writing of the image capture signals on the three frame memories (not shown) and stops the output of the still image created at the timing of the time series number 3 shown in Fig. 20. Then, the synchronization circuit 53 sequentially writes the image capture signals including the G2 signal, the fluorescence signal, and the R2 signal on the three frame memories (not shown) provided inside of the circuit as synchronization memories, simultaneously reads the written image capture signals and outputs the synchronized image capture signals. Thus, a self-fluorescent image is displayed as a moving image.

That is, with the above-described control performed by the processor 6, in the case that the user sets the observation mode switching time to the smallest value, the time necessary for the observation mode switching may be minimized, and at the time of observation mode switching, the still image other than the still images having significant noise may be obtained as the freeze image.

The set value of the observation mode switching time is not limited to the desired value set by the user, but, for example, the set value may be set by the control circuit 40 based on information about the model of the endoscope or the configuration of the image capturing section, or the like written on the identification information circuit 43 or a scope ID memory 48.

Specifically, based on the information about the model of the endoscope or the configuration of the image capturing section, or the like written on the identification information circuit 43 or the scope ID memory 48, for example, in a case that the control circuit 40 detects that the image capturing section of the electronic endoscope 2 is the image capturing section 30 that has two CCDs, the control circuit 40 sets the set value of the observation mode switching time to a relatively large value. Further, based on the information written on the identification information circuit 43 or the scope ID memory 48, for example, in a case that the control circuit 40 detects that the image capturing section of the electronic endoscope 2 is the image capturing section 30A that has one CCD, the control circuit 40 sets the set value of the observation mode switching time to a relatively small value.

The set value of the observation mode switching time is not limited to the above-described desired value of the user or the value set by the control circuit 40, but, for example, the set value may be a fixed value written on the identification information circuit 43 as the information storage means or the scope ID memory 48 as the information storage means.

The color shift detection circuit 47, in the above-described pre-freeze processing, may perform the following processing.

For example, in the time series number 5 shown in Fig. 21, a case that the observation mode in the endoscope device 1 is changed from one observation mode to another observation mode will be described. The color shift values shown in Fig. 21 are expressed in hexadecimal numerals.

In such a case, the color shift detection circuit 47 invalidates the freeze instruction issued at the timing of the time series numbers 5 and 6 shown in Fig. 21 that is the timing right after the observation mode in the endoscope device 1 is switched from the one observation mode to the other observation mode, and does not execute the pre-freeze processing.

In a case that the processing level value in the pre-freeze processing is set to 6, in addition to the above-described time series numbers 5 and 6, as an inoperative time of the freeze instruction in accordance with the above processing level, for example, the color shift detection circuit 47 invalidates a freeze instruction issued between the time series number 7 and the time series number 35. Then, at the timing of F3 shown in Fig. 21, that is, in a case that the freeze instruction is issued at the time series number 36, the color shift detection circuit 47 detects a least color shifted image capture signal out of the image capture signals written on the memory section 39 in the time period between the time series number 7 and the time series number 36, and then executes the pre-freeze processing such that the still image according to the image capture signal is displayed on the monitor 7 as the freeze image. Thus, among the image capture signals written on the memory section 39 in the time period between the time series number 7 and the time series number 36, the still image according to the least color shifted image capture signal, for example, the image of the time series number 34 shown in Fig. 21 is displayed on the monitor 7, as the freeze image.

In a case that the processing level value in the pre-freeze processing is set to 7, in addition to the above-described time series numbers 5 and 6, as an inoperative time of the freeze instruction in accordance with the above processing level, for example, the color shift detection circuit 47 invalidates a freeze instruction issued between the time series number 7 and the time series number 62. Then, at the timing of F4 shown in Fig. 21, that is, in a case that the freeze instruction is issued at the time series number 63, the color shift detection circuit 47 detects a least color shifted image capture signal out of the image capture signals written on the memory section 39 in the time period between the time series number 7 and the time series number 62, and executes the pre-freeze processing such that the still image according to the image capture signal is displayed on the monitor 7 as the freeze image. Thus, among the image capture signals written on the memory section 39 in the time period between the time series number 7 and the time series number 62, the still image according to the least color shifted image capture signal, for example, the image of the time series number 34 shown in Fig. 21 is displayed on the monitor 7, as the freeze image.

In the above-described pre-freeze processing, the color shift detection circuit 47 is not limited to set the inoperative time of the freeze instruction depending on the processing level of the pre-freeze processing. The color shift detection circuit 47, depending on the processing level, may set the color shift value of the image capture signal in a time series number not to be pre-freeze processed to a maximum value, and not extract as the freeze image.

In the above-described pre-freeze processing, the color shift detection circuit 47 is not limited to set the inoperative time to be set depending on the processing level of the pre-freeze processing only to the freeze instruction, for example, the inoperative time may be similarly set with respect to each instruction other than the freeze instruction. Specifically, the color shift detection circuit 47 that has the functions as the image operation invalidation means and image operation invalidation release means may set the inoperative time in addition to the above-described freeze instruction as each instruction with respect to the image operation performed in any of the keyboard 9, the scope switch 10, and the front panel 55 of the processor 6, with respect to a release instruction, an image emphasis instruction, a color conversion instruction, an enlarged display instruction, an observation mode switching instruction, and a comment input instruction, depending on the processing level in the pre-freeze processing. For example, in a case that the endoscope device 1 has an air feeding function, in the above-described pre-freeze processing, the color shift detection circuit 47, with respect to an air feeding instruction performed in the scope switch 10 or the like, may not set the inoperative time depending on the processing level of the pre-freeze processing.

Further, in a case that without setting the inoperative time depending on the processing level of the pre-freeze processing, only the freeze instruction issued right after the observation mode in the endoscope device 1 is switched from the one observation mode to the other observation mode, that is, only the freeze instruction issued at the timing of the time series numbers 5 and 6 shown in Fig. 21 is to be invalidated, the color shift detection circuit 47 performs the following processing as processing included in the pre-freeze processing.

In a case that the processing level value in the pre-freeze processing is set to 7, and the freeze operation is executed at a timing of F4 shown in Fig. 21, that is, at the timing of the time series number 63, based on the image capture signals written on the memory section 39 at the time between the time series number 7 and the time series number 63, as shown in Fig. 22, the color shift detection circuit 47 extracts, for example, five sheets of still images in order of the image less color shifted.

Then, the color shift detection circuit 47, for example, instructs the control circuit 40 to create still images of the five sheets of still images and display the five sheets of still images on the monitor 7 such that the user may select a desired freeze image out of the extracted five sheets of still images.

Based on the above-described instruction performed by the color shift detection circuit 47 to the control circuit 40, on the monitor 7, for example, as shown in Fig. 22, out of the extracted five sheets of still images, a least color shifted image of the time series number 34 is displayed first. Further, based on the above-described instruction performed by the color shift detection circuit 47 to the control circuit 40, on the monitor 7, for example, as shown in Fig. 22, the five sheets of still images are sequentially displayed one by one in a state that a desired freeze image cab be selected by operating the keyboard 9 or the like.

Then, by the user, for example, in a case that an image of the time series number 33 is selected, the image of the time series number 33 is displayed on the monitor 7 as the freeze image.

That is, with the color shift detection circuit 47, in the above-described pre-freeze processing, in the case that image capture signals in the one observation mode are written more than sheets of images corresponding to the processing level value in the pre-freeze processing, enables the selection of the freeze images by the user. Thus, the user may obtain the desired less color shifted image as the freeze image. The order of display of the each still image displayed such that a desired freeze image may be selected is not limited to the time series order as shown in Fig. 22, but may be an order of less color shifted.

In a case that the processing level value in the pre-freeze processing is set to 7, and the freeze operation is executed at a timing of F3 shown in Fig. 21, that is, at the timing of the time series number 36, based on the image capture signals written on the memory section 39 at the time between the time series number 7 and the time series number 63, for example, as shown in Fig. 23, the color shift detection circuit 47 extracts an image of the time series number 34 as the least color shifted image and displays the image of the time series number 34 as the freeze image on the monitor 7. In such a processing, images according to image capture signals written on the memory section 39 before the time series number 6 are not suitable for the freeze image. Accordingly, these images are not extracted by the color shift detection circuit 47.

That is, the color shift detection circuit 47, in the above-described pre-freeze processing, in the case that image capture signals in the one observation mode are not written more than sheets of images corresponding to the processing level value in the pre-freeze processing, invalidates the selection of the freeze images by the user and displays the least color shifted image as the freeze image on the monitor 7. The color shift detection circuit 47, in the case that image capture signals in the one observation mode are not written more than sheets of images corresponding to the processing level value in the pre-freeze processing, even if the freeze operation is sequentially performed, as described above, the selection of the freeze image by the user is invalidated.

As described above, the endoscope device 1 according to the embodiment may output the still image suitable for recording in the case that the one observation mode is switched to the other observation mode.

It is to be understood that in the endoscope device 1 according to the embodiment, the configuration may be variously modified without departing from the spirit of the present invention.

**Claims**

1. An image processing device comprising:

   image capturing means for capturing an image of a subject and outputting an image capture signal based on the captured image of the subject;
   one or a plurality of storage means for storing the image capture signal outputted from the image capturing means;
   writing signal generation means for outputting to the storage means a writing signal for writing the image capture signal onto the storage means;
   switching signal generation means for outputting to at least one of the image capturing means and the storage means a switching signal for switching between a first observation mode for creating a first observation image based on the image capture signal outputted from the image capturing means and a second observation mode for creating a second observation image different from the first observation image based on the image capture signal outputted from the image capturing means;
   image operation means for performing an instruction about an operation with respect to at least one of the first observation image and the second observation image;
   image operation invalidation means for setting an inoperative time for invalidating the instruction about the operation with respect to the one observation image based on the switching signal within a predetermined period of time; and
   image operation invalidation release means for releasing the invalidation after the switching signal is outputted and the inoperative time has passed.

2. An image processing device comprising:

   image capturing means for capturing an image of a subject and outputting an image capture signal based on the captured image of the subject;
   one or a plurality of storage means for storing the image capture signal outputted from the image capturing means;
   writing signal generation means for outputting to the storage means a writing signal for writing the image capture signal onto the storage means;
   switching signal generation means for outputting to at least one of the image capturing means and the storage means a switching signal for switching between a first observation mode for creating a first observation image based on the image capture signal outputted from the image capturing means and a second observation mode for creating a second observation image different from the first observation image based on the image capture signal outputted from the image capturing means;
   writing forbidding means for stopping the writing of the image capture signal onto the storage means by stopping the output of the writing signal according to the switching signal; and
   writing forbiddance release means for releasing the stop of the writing of the image capture signal onto the storage means by resuming the output of the writing signal to the storage means after the switching signal is outputted and a predetermined period of time has passed.

3. The image processing device according to Claim 2, further comprising:

   freeze image creation means having the storage means, the freeze image creation means being configured to create a still image based on the image capture signal written on the storage means; and
   freeze instruction means for performing a freeze instruction for creating the still image to the freeze image creation means;

   wherein the freeze image creation means invalidates the freeze instruction performed in the freeze instruction means for the predetermined period of time.

4. The image processing device according to Claim 2, further comprising:

   observation mode switching time setting means for setting the predetermined period of time.

5. The image processing device according to Claim 2, further comprising:

   information storage means on which certain information about at least a configuration of the image capturing means is written;

wherein the predetermined period of time is set based on the certain information.

6. The image processing device according to Claim 3, wherein the freeze image creation means further performs processing for extracting a plurality of still images including a least color shifted still image out of still images according to the image capture signal written on the storage means.

7. The image processing device according to Claim 1, further comprising:

freeze image creation means having the storage means, the freeze image creation means being configured to perform processing for extracting a plurality of still images including a least color shifted still image out of still images according to the image capture signal written on the storage means; and
freeze instruction means for performing a freeze instruction for creating the plurality of still images extracted by the freeze image creation means to the freeze image creation means;

wherein the freeze image creation means invalidates the processing in a case that the freeze instruction is performed in the freeze instruction means within the predetermined period of time except for the inoperative time.

8. The image processing device according to Claim 1, wherein in the first observation image created in the first observation mode and the second observation image created in the second observation mode, one observation image denotes an image substantially similar to an image of the subject being observed with the naked eye, and another observation image denotes an image corresponding to an image of fluorescence generated by the subject.

9. The image processing device according to Claim 2, wherein in the first observation image created in the first observation mode and the second observation image created in the second observation mode, one observation image denotes an image substantially similar to an image of the subject being observed with the naked eye, and another observation image denotes an image corresponding to an image of fluorescence generated by the subject.

10. The image processing device according to Claim 3, wherein in the first observation image created in the first observation mode and the second observation image created in the second observation mode, one observation image denotes an image substantially similar to an image of the subject being observed with the naked eye, and another observation image denotes an image corresponding to an image of fluorescence generated by the subject.

11. The image processing device according to Claim 4, wherein in the first observation image created in the first observation mode and the second observation image created in the second observation mode, one observation image denotes an image substantially similar to an image of the subject being observed with the naked eye, and another observation image denotes an image corresponding to an image of fluorescence generated by the subject.

12. The image processing device according to Claim 5, wherein in the first observation image created in the first observation mode and the second observation image created in the second observation mode, one observation image denotes an image substantially similar to an image of the subject being observed with the naked eye, and another observation image denotes an image corresponding to an image of fluorescence generated by the subject.

13. The image processing device according to Claim 6, wherein in the first observation image created in the first observation mode and the second observation image created in the second observation mode, one observation image denotes an image substantially similar to an image of the subject being observed with the naked eye, and another observation image denotes an image corresponding to an image of fluorescence generated by the subject.

14. The image processing device according to Claim 7, wherein in the first observation image created in the first observation mode and the second observation image created in the second observation mode, one observation image denotes an image substantially similar to an image of the subject being observed with the naked eye, and another observation image denotes an image corresponding to an image of fluorescence generated by the subject.

15. The image processing device according to Claim 1, further comprising:

an endoscope including an elongated insertion portion;

wherein the image capturing means is provided in a tip part of the insertion portion.

**16.** The image processing device according to Claim 2, further comprising:

an endoscope including an elongated insertion portion;

wherein the image capturing means is provided in a tip part of the insertion portion.

**17.** The image processing device according to Claim 3, further comprising:

an endoscope including an elongated insertion portion;

wherein the image capturing means is provided in a tip part of the insertion portion.

**18.** The image processing device according to Claim 4, further comprising:

an endoscope including an elongated insertion portion;

wherein the image capturing means is provided in a tip part of the insertion portion.

**19.** The image processing device according to Claim 5, further comprising:

an endoscope including an elongated insertion portion;

wherein the image capturing means is provided in a tip part of the insertion portion.

**20.** The image processing device according to Claim 6, further comprising:

an endoscope including an elongated insertion portion;

wherein the image capturing means is provided in a tip part of the insertion portion.

**21.** The image processing device according to Claim 7, further comprising:

an endoscope including an elongated insertion portion;

wherein the image capturing means is provided in a tip part of the insertion portion.

EP 1 844 697 A1

# FIG.1

LIGHT SOURCE SECTION

VIDEO PROCESSING BLOCK

IMAGE PROCESSING BLOCK

MONITOR IMAGE PHOTOGRAPHING DEVICE

IMAGE FILING DEVICE

KEYBOARD

21

FIG.2

# FIG.3

# FIG.4

# FIG.5

# FIG.6

# FIG.7

80a

80c

TRANSMITTANCE

400   500   600   700   800   900   1000   (nm)

WAVELENGTH

# FIG.8

80b

TRANSMITTANCE

400   500   600   700   800   900   1000   (nm)

WAVELENGTH

# FIG.9

32

TRANSMITTANCE

400   450   500   550   600   650   700   (nm)

WAVELENGTH

# FIG.10

```
MENU 1/2

DISPLAY SIZE            : FULL HEIGHT

FLUORESCENCE
OBSERVATION            : MEDIUM
DISPLAY SIZE

INTEREST REGION        : LARGE

IHb RANGE              : Normal

IHb AVERAGE VALUE      : ON
```

# FIG.11

# FIG.12

EP 1 844 697 A1

# FIG.13

```
                    ( START )
                        |
                        v
              ┌─────────────────────┐
              │      IS MODE        │  S1
              │     SWITCHED        │
           ◇  FROM ONE OBSERVATION  ◇──── NO ──┐
              │  MODE TO ANOTHER    │          │
              │    OBSERVATION      │          │
              │       MODE?         │          │
              └─────────────────────┘          │
                        |                       │
                       YES                      │
                        v                       │
        ┌──────────────────────────┐           │
        │    OUTPUT STILL IMAGE     │─── S2     │
        └──────────────────────────┘           │
                        |                       │
                        v                       │
        ┌──────────────────────────┐           │
        │  DRIVE ANOTHER CCD WHILE  │─── S3     │
        │ STOPPING DRIVE OF ONE CCD │           │
        └──────────────────────────┘           │
                        |                       │
                        v                       │
        ┌──────────────────────────┐           │
        │  CHANGE ROTATION SPEED OF │─── S4     │
        │     ROTATION FILTER 27    │           │
        └──────────────────────────┘           │
                        |                       │
                        v                       │
        ┌──────────────────────────┐           │
        │     START COUNT OF        │─── S5     │
        │   PREDETERMINED TIME      │           │
        └──────────────────────────┘           │
                        |                       │
                        v<──────────┐           │
              ┌─────────────────┐   │           │
              │       HAS       │ S6│           │
           ◇  PREDETERMINED     ◇── NO          │
              │  TIME PASSED?   │               │
              └─────────────────┘               │
                        |                       │
                       YES                      │
                        v                       │
        ┌──────────────────────────┐           │
        │  STOP OUTPUT OF STILL IMAGE│── S7     │
        └──────────────────────────┘           │
                        |                       │
                        v                       │
        ┌──────────────────────────┐           │
        │   PERFORM PROCESSING      │           │
        │    APPROPRIATE FOR        │           │
        │  OUTPUT OF MOVING IMAGE   │─── S8     │
        │ WHILE RESUME OUTPUT OF    │           │
        │     MOVING IMAGE          │           │
        └──────────────────────────┘           │
                        |                       │
                        v<──────────────────────┘
                    (  END  )
```

28

# FIG.14

| TIME SERIES NUMBER | 1 | 2 | 3 | 4 | 5-10 | 11 | 12 | 13 | 14 | 15 | 16 | 17 | 18 | 19 | 20 | 21 |
|---|---|---|---|---|---|---|---|---|---|---|---|---|---|---|---|---|
| CCD | 1 | 1 | 1 | 1 | 2 | 2 | 2 | 2 | 2 | 2 | 2 | 2 | 2 | 2 | 2 | 2 |
| ROTATION FILTER | B0 | R1 | G1 | B1 | X | R2 | R2 | G2 | G2 | B2 | B2 | R3 | R3 | G3 | G3 | B3 |
| MEMORY PART WRITING | B0 | R1 | G1 | B1 | R2··· | – | R2 | – | G2 | – | B2 | – | R3 | | | |
| R MEMORY READOUT | R0 | – | R1 | R1 | – ··· | R1' | – | R2 | R2 | R2 | R2 | R2 | – | | | |
| G MEMORY READOUT | G0 | G0 | – | G1 | G1··· | G1' | G1' | G1' | – | G2 | G2 | G2 | G2 | G2 | | |
| B MEMORY READOUT | – | B0 | B0 | – | B1··· | B1' | B1' | B1' | B1' | B1' | – | B2 | B2 | B2 | B2 | B2 |
| NOISE GENERATION (△) | | | | | △ | | | | | | | | | | | |
| MOVING IMAGE/STILL IMAGE | MOVING | MOVING | MOVING | STILL | STILL | MOVING | MOVING | MOVING | MOVING | MOVING | MOVING | MOVING | MOVING | MOVING | MOVING | MOVING |
| FREEZE INSTRUCTION | | | | | | | F1 | | | | | | | | | F2 |

EP 1 844 697 A1

# FIG.15

MENU 2/2

FREEZE LEVEL          : 4

COLOR SHIFT          : PREFREEZE
DETECTION

CHARACTER DISPLAY : FULL

PHOTOMETRY          : AVERAGE

OBSERVATION MODE  : 2
SWITCHING TIME

# FIG.16

```
                    ( START )
                        │
                        ▼
              ┌─────────────────────┐
         ╱    IS MODE          ╲  ⌐S11
        ╱     SWITCHED          ╲
       ╱   FROM ONE OBSERVATION  ╲      NO
       ╲    MODE TO ANOTHER      ╱──────────────────┐
        ╲    OBSERVATION        ╱                   │
         ╲      MODE?          ╱                    │
              └──────┬──────┘                       │
                   │ YES                            │
                   ▼                                │
              ┌───────────────┐ ⌐S12                │
         ╱   IS EXPOSURE    ╲        NO             │
         ╲  TIME CHANGED?   ╱──────────────┐        │
              └─────┬─────┘                │        │
                  │ YES    ⌐S13            │  ⌐S14  │
                  ▼                        ▼        │
      ┌────────────────────────┐  ┌────────────────────────┐
      │  SET PERIOD OF TIME FOR │  │  SET PERIOD OF TIME FOR │
      │   INVALIDATING FREEZE   │  │   INVALIDATING FREEZE   │
      │ INSTRUCTION FOR 3 SECONDS│ │INSTRUCTION FOR 0.1 SECOND│
      └───────────┬────────────┘  └───────────┬────────────┘
                  │◄────────────────────────────┘
                  ▼
      ┌────────────────────────┐
      │   INVALIDATE FREEZE     │── S15
      │      INSTRUCTION        │
      └───────────┬────────────┘
                  ▼
      ┌────────────────────────┐
      │ START COUNT OF TIME PASSED│
      │ SINCE THE ONE OBSERVATION │── S16
      │ MODE HAS SWITCHED TO THE  │
      │  OTHER OBSERVATION MODE   │
      └───────────┬────────────┘
                  ▼◄──────────────────────────┐
         ╱    HAS         ╲  ⌐S17             │
        ╱  PERIOD OF TIME  ╲                   │
       ╱  FOR INVALIDATING  ╲      NO          │
       ╲ FREEZE INSTRUCTION ╱──────────────────┘
        ╲     PASSED?      ╱
              └────┬─────┘
                 │ YES
                 ▼
      ┌──────────────────────────────┐
      │ VALIDATE FREEZE INSTRUCTION   │── S18
      └──────────────┬───────────────┘
                     ▼◄───────────────────────┐
                 ( END )
```

# FIG.17

```
                    ( START )
                        │
                        ▼
        ┌───────────────────────────────┐
        │   HOLD FIRST PROCESSING LEVEL │── S21
        │           VALUE               │
        └───────────────────────────────┘
                        │
                        ▼
        ┌───────────────────────────────┐
        │  SET SECOND PROCESSING LEVEL  │── S22
        │  VALUE AND PERIOD OF TIME FOR │
        │ INVALIDATING FREEZE INSTRUCTION│
        └───────────────────────────────┘
                        │◄──────────────────────┐
                        ▼         S23           │
                    ╱ IS MODE ╲                 │
                  ╱  SWITCHED FROM  ╲    NO      │
                 ◄ ONE OBSERVATION MODE TO ──────┘
                  ╲ ANOTHER OBSERVATION ╱
                    ╲   MODE?  ╱
                        │ YES
                        ▼
        ┌───────────────────────────────┐
        │  START COUNT OF TIME PASSED   │── S24
        │  SINCE THE ONE OBSERVATION    │
        │  MODE HAS SWITCHED TO THE     │
        │  OTHER OBSERVATION MODE       │
        └───────────────────────────────┘
                        │
                        ▼
        ┌───────────────────────────────┐
        │  INCREASE SECOND PROCESSING   │── S25
        │   LEVEL VALUE EVERY TIME      │
        │ PREDETERMINED TIME HAS PASSED │
        └───────────────────────────────┘
                        │
                        ▼
        ┌───────────────────────────────┐
        │      FREEZE INSTRUCTION       │── S26
        └───────────────────────────────┘
                        │
                        ▼         S27
                    ╱ FIRST ╲
                  ╱ PROCESSING LEVEL ╲   NO
                 ◄ VALUE > SECOND PROCESSING ──────────┐
                  ╲ LEVEL VALUE? ╱                     │
                        │ YES      S28                 │ S29
                        ▼                              ▼
        ┌───────────────────────────┐   ┌───────────────────────────┐
        │   EXECUTE PREFREEZE       │   │   EXECUTE PREFREEZE        │
        │ PROCESSING BASED ON FIRST │   │ PROCESSING BASED ON SECOND│
        │  PROCESSING LEVEL VALUE   │   │  PROCESSING LEVEL VALUE   │
        └───────────────────────────┘   └───────────────────────────┘
                        │◄──────────────────────────────┘
                        ▼
                    ( END )
```

# FIG.18

| TIME SERIES NUMBER | 1 | 2 | 3 | 4 | 5-10 | 11 | 12 | 13 | 14 | 15 | 16 | 17 |
|---|---|---|---|---|---|---|---|---|---|---|---|---|
| CCD | 1 | 1 | 1 | 1 | 2 | 2 | 2 | 2 | 2 | 2 | 2 | 2 |
| ROTATION FILTER | R1 | G1 | B1 | - | X | R8 | R8 | G8 | G8 | B8 | B8 | R9 |
| SYNCHRONIZATION CIRCUIT R WRITING | R1 | - | - | - | - | R8 | R8 | - | - | - | - | R9 |
| SYNCHRONIZATION CIRCUIT G WRITING | | G1 | - | - | - | - | - | G8 | G8 | - | - | - |
| SYNCHRONIZATION CIRCUIT B WRITING | | - | B1 | - | - | - | - | - | | B8 | B8 | - |
| | | | | | | | | | | | | |
| SYNCHRONIZATION CIRCUIT R READOUT | | R1 | R1 | R1 | R1 | - | - | R8 | R8 | R8 | R8 | - |
| SYNCHRONIZATION CIRCUIT G READOUT | G0 | - | G1 | G1 | G1 | G7 | G7 | - | - | G8 | G8 | G8 |
| SYNCHRONIZATION CIRCUIT B READOUT | B0 | B0 | - | B1 | B1 | B7 | B7 | B7 | B7 | - | - | B8 |
| | | | | | | | | | | | | |
| DISPLAY R | R1 | R1 | R1 | R1 | R1 | R8 | R8 | R8 | R8 | R8 | R8 | R9 |
| DISPLAY G | G0 | G1 | G1 | G1 | G1 | G7 | G7 | G8 | G8 | G8 | G8 | G8 |
| DISPLAY B | B0 | B0 | B1 | B1 | B1 | B7 | B7 | B7 | B7 | B8 | B8 | B8 |
| MOVING IMAGE/STILL IMAGE | MOVING | MOVING | MOVING | STILL | STILL | MOVING | MOVING | MOVING | MOVING | MOVING | MOVING | MOVING |

EP 1 844 697 A1

# FIG.19

| TIME SERIES NUMBER | 1 | 2 | 3 | 4 | 5-21 | 22 | 23 | 24 | 25 | 26 | 27 | 28 |
|---|---|---|---|---|---|---|---|---|---|---|---|---|
| CCD | 1 | 1 | 1 | 1 | 2 | 2 | 2 | 2 | 2 | 2 | 2 | 2 |
| ROTATION FILTER | R1 | G1 | B1 | R2 | X | R8 | R8 | G8 | G8 | B8 | B8 | R9 |
| SYNCHRONIZATION CIRCUIT R WRITING | R1 | - | - | - | - | R8 | R8 | - | - | - | - | R9 |
| SYNCHRONIZATION CIRCUIT G WRITING | - | G1 | - | - | - | - | - | G8 | G8 | - | - | - |
| SYNCHRONIZATION CIRCUIT B WRITING | - | - | B1 | - | - | - | - | - | | B8 | B8 | - |
| | | | | | | | | | | | | |
| SYNCHRONIZATION CIRCUIT R READOUT | R1 | R1 | R1 | R1 | R1 | R8 | R8 | R8 | R8 | R8 | R8 | - |
| SYNCHRONIZATION CIRCUIT G READOUT | G0 | G1 | G1 | G1 | G1 | G1 | G1 | B8 | B8 | G8 | G8 | G8 |
| SYNCHRONIZATION CIRCUIT B READOUT | B0 | B0 | B1 | B1 | B1 | B1 | B1 | B1 | B1 | B8 | B8 | B8 |
| | | | | | | | | | | | | |
| DISPLAY R | R1 | R1 | R1 | R1 | R1 | R8 | R8 | R8 | R8 | R8 | R8 | R9 |
| DISPLAY G | G0 | G1 | G1 | G1 | G1 | G1 | G1 | G8 | G8 | G8 | G8 | G8 |
| DISPLAY B | B0 | B0 | B1 | B1 | B1 | B1 | B1 | B1 | B1 | B8 | B8 | B8 |
| MOVING IMAGE/STILL IMAGE | MOVING | MOVING | MOVING | STILL | STILL | MOVING | MOVING | MOVING | MOVING | MOVING | MOVING | MOVING |

EP 1 844 697 A1

## FIG.20

| TIME SERIES NUMBER | 1 | 2 | 3 | 4 | 5-12 | 13 | 14 | 15 | 16 | 17 | 18 | 19 |
|---|---|---|---|---|---|---|---|---|---|---|---|---|
| CCD | 1 | 1 | 1 | 1 | 2 | 2 | 2 | 2 | 2 | 2 | 2 | 2 |
| ROTATION FILTER | R1 | G1 | B1 | R2 | X | R5 | R5 | G5 | G5 | B5 | B5 | R6 |
| SYNCHRONIZATION CIRCUIT R WRITING | R1 | - | - | - | - | R5 | R5 | - | - | - | - | R6 |
| SYNCHRONIZATION CIRCUIT G WRITING | - | G1 | - | - | - | - | - | G5 | G5 | - | - | - |
| SYNCHRONIZATION CIRCUIT B WRITING | - | - | B1 | - | - | - | - | - | | B5 | B5 | - |
| | | | | | | | | | | | | |
| SYNCHRONIZATION CIRCUIT R READOUT | R1 | R1 | R1 | R1 | R1 | R5 | R5 | R5 | R5 | R5 | R5 | R6 |
| SYNCHRONIZATION CIRCUIT G READOUT | G0 | G1 | G1 | G1 | G1 | G1 | G5 | B5 | B5 | G5 | G5 | G5 |
| SYNCHRONIZATION CIRCUIT B READOUT | B0 | B0 | B1 | B1 | B1 | B1 | B1 | B1 | B1 | B5 | B5 | B5 |
| | | | | | | | | | | | | |
| DISPLAY R | R1 | R1 | R1 | R1 | R1 | R5 | R5 | R5 | R5 | R5 | R5 | R6 |
| DISPLAY G | G0 | G1 | G1 | G1 | G1 | G1 | G1 | G5 | G5 | G5 | G5 | G5 |
| DISPLAY B | B0 | B0 | B1 | B1 | B1 | B1 | B1 | B1 | B1 | B5 | B5 | B5 |
| MOVING IMAGE/STILL IMAGE | MOVING | MOVING | MOVING | STILL | STILL | MOVING | MOVING | MOVING | MOVING | MOVING | MOVING | MOVING |

EP 1 844 697 A1

## FIG.21

| TIME SERIES NUMBER | 1 | 2 | 3 | 4 | 5 | 6 | 7 | ... | 33 | 34 | 35 | 36 | ... | 61 | 62 | 63 | ... |
|---|---|---|---|---|---|---|---|---|---|---|---|---|---|---|---|---|---|
| MEMORY PART WRITING | B0 | R1 | G1 | B1 | R2 | G2 | B2 | ... | G11 | B11 | R12 | G12 | ... | B20 | R21 | G21 | ... |
| R MEMORY READOUT | R0 | - | R1 | R1 | - | R2 | R2 | ... | R11 | R11 | - | R12 | ... | R20 | - | R21 | ... |
| G MEMORY READOUT | G0 | G0 | - | G1 | G1 | - | G2 | ... | - | G11 | G11 | - | ... | G20 | G20 | - | ... |
| B MEMORY READOUT | - | B0 | B0 | - | B1 | B1 | - | ... | B10 | - | B11 | B11 | ... | - | B20 | B20 | ... |
| COLOR SHIFT VALUE | 55 | 5E | 5F | 5D | 5E | 55 | 5C | ... | 58 | 56 | 5A | 5B | ... | 57 | 59 | 57 | ... |
| OBSERVATION MODE | A | A | A | A | B | B | B | ... | B | B | B | B | ... | B | B | B | B |
| FREEZE INSTRUCTION | | | | | | | | | | | | F3 | | | | F4 | |

# FIG.22

SELECTION

TIME SERIES NUMBER 33 → TIME SERIES NUMBER 34 → TIME SERIES NUMBER 61 → TIME SERIES NUMBER 62 → TIME SERIES NUMBER 63

SELECTION    SELECTION    SELECTION    SELECTION

⇧

TIME SERIES NUMBER 33    TIME SERIES NUMBER 34    TIME SERIES NUMBER 61    TIME SERIES NUMBER 62    TIME SERIES NUMBER 63

⇧ EXTRACTION

TIME SERIES NUMBER 7 → TIME SERIES NUMBER 8 ~ 62 → TIME SERIES NUMBER 63

39

# FIG.23

7

DISPLAY

**TIME SERIES
NUMBER 34**

**IMAGES OTHER THAN
IMAGE TO BE EXTRACTED**   ⇑ **EXTRACTION**

**TIME SERIES
NUMBER 6**

**TIME SERIES
NUMBER 7**

**TIME SERIES
NUMBER 36**

*39*

**European Patent** **PARTIAL EUROPEAN SEARCH REPORT** **Application Number**
**Office** which under Rule 45 of the European Patent Convention EP 07 00 5680
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | EP 1 491 132 A (OLYMPUS CORP [JP]) 29 December 2004 (2004-12-29)  * paragraphs [0052] - [0054] * * paragraphs [0080] - [0091] * * paragraphs [0127] - [0141] * * figure 1 * | 1,2,4,8, 9,11,15, 16,18 | INV. A61B1/05 |
| Y | | 3,5-7, 10, 12-14, 17,19-21 | |
| Y | EP 1 302 152 A (OLYMPUS OPTICAL CO [JP]) 16 April 2003 (2003-04-16)  * paragraphs [0054], [0055] * ----- | 3,6,7, 10,13, 14,17, 20,21 | |
| Y | EP 1 484 001 A (OLYMPUS CORP [JP]) 8 December 2004 (2004-12-08) * paragraphs [0058] - [0066] * ----- -/-- | 6,7,13, 14,20,21 | |

**TECHNICAL FIELDS SEARCHED (IPC)**

A61B

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 5 September 2007 | Bengtsson, Johan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

**European Patent Office**

**PARTIAL EUROPEAN SEARCH REPORT**

Application Number

EP 07 00 5680

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| Y | EP 1 258 221 A (OLYMPUS OPTICAL CO [JP]) 20 November 2002 (2002-11-20) * paragraphs [0053], [0214] * ----- | 5,12,19 | |
| X | US 5 387 928 A (NISHIMURA SHIGERU [JP]) 7 February 1995 (1995-02-07) * column 4, line 3 - column 5, line 16 * * figure 1 * ----- | 1,2,15, 16 | |
| P,X | JP 2006 340855 A (OLYMPUS MEDICAL SYSTEMS CORP) 21 December 2006 (2006-12-21)  * the whole document * ----- | 1-4, 6-11, 13-18, 20,21 | |

TECHNICAL FIELDS SEARCHED (IPC)

EPO FORM 1503 03.82 (P04C10)

Claim(s) searched incompletely:
    1-21

Claim(s) not searched:
        -

Reason for the limitation of the search:

Present claims 1-21 relate to a generic image processing device. Support within the meaning of Article 84 EPC and/or disclosure within the meaning of Article 83 EPC is to be found, however, for only a very narrow application, namely electronic endoscopes. In the present case, the claims so lack support, and the application so lacks disclosure, that a meaningful search over the whole of the claimed scope is impossible. Consequently, the search has been carried out for those parts of the claims which appear to be supported and disclosed, namely those parts relating to an electronic endoscope comprising the features claimed in claims 1-21.

## ANNEX TO THE EUROPEAN SEARCH REPORT
## ON EUROPEAN PATENT APPLICATION NO.

EP 07 00 5680

This annex lists the patent family members relating to the patent documents cited in the above-mentioned European search report.
The members are as contained in the European Patent Office EDP file on
The European Patent Office is in no way liable for these particulars which are merely given for the purpose of information.

05-09-2007

| Patent document cited in search report | | Publication date | Patent family member(s) | | Publication date |
|---|---|---|---|---|---|
| EP 1491132 | A | 29-12-2004 | CN | 1578471 A | 09-02-2005 |
| | | | JP | 2005013611 A | 20-01-2005 |
| | | | US | 2004263643 A1 | 30-12-2004 |
| EP 1302152 | A | 16-04-2003 | WO | 0207588 A1 | 31-01-2002 |
| EP 1484001 | A | 08-12-2004 | AU | 2003221384 A1 | 22-09-2003 |
| | | | CN | 1638687 A | 13-07-2005 |
| | | | WO | 03075752 A1 | 18-09-2003 |
| | | | JP | 2003334162 A | 25-11-2003 |
| | | | US | 2005078175 A1 | 14-04-2005 |
| EP 1258221 | A | 20-11-2002 | US | 2002175993 A1 | 28-11-2002 |
| US 5387928 | A | 07-02-1995 | NONE | | |
| JP 2006340855 | A | 21-12-2006 | NONE | | |

EPO FORM P0459

For more details about this annex : see Official Journal of the European Patent Office, No. 12/82

**EP 1 844 697 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005013611 A